# EUROPEAN PATENT APPLICATION

(11) **EP 3 791 897 A1**
(43) Date of publication of application: **17.03.2021**
(21) Application number: 20190315.0
(22) Date of filing: 24.02.2017
(51) Int. Cl.: A61K 47/55, A61P 35/00, C07D 249/08, C07D 401/14, C07D 471/04

(54) **HSP90 INHIBITOR DRUG CONJUGATES**

(30) Priority: 29.02.2016 US 201662301052 P
(62) Divisional of application: 17760503.7
(71) Applicant: Madrigal Pharmaceuticals, Inc., West Conshohocken, Pennsylvania 19428 (US)
(72) Inventor: Chimmanamada, Dinesh U., Arlington, Massachusetts 02474 (US); Kostik, Elena, Arlington, Massachusetts 02474 (US); Vutukuri, Dharma, Woburn, Massachusetts 01801 (US); Ying, Weiven, Lexington, Massachusetts 02421 (US); Zhang, Junyi, Lexington, Massachusetts 02421 (US)
(74) Representative: Hoffmann Eitle

(57) **Abstract**

The application concerns an Hsp90 drug conjugate (HDC) comprising an Hsp90 ligand or prodrug thereof, a linker moiety and a payload moiety, wherein the payload moiety is an ERK kinase inhibitor.

## Description

### REFERENCE TO RELATED APPLICATIONS

The present application claims priority to U.S. Provisional Application No. 62/301,052, filed February 29, 2016, entitled HSP90 Inhibitor Drug Conjugates, the contents of which are herein incorporated by reference in their entirety.

### BACKGROUND OF THE INVENTION

Many diseases and disorders are characterized by the presence of high levels of certain proteins in specific types of cells. In some cases, the presence of these high levels of protein is caused by overexpression. Historically, some of these proteins have been useful targets for therapeutic molecules or used as biomarkers for the detection of disease. One class of overexpressed intracellular protein that has been recognized as a useful therapeutic target is known as the heat shock proteins.

Heat shock proteins (HSPs) are a class of proteins that are up-regulated in response to elevated temperature and other environmental stresses, such as ultraviolet light, nutrient deprivation, and oxygen deprivation. HSPs have many known functions, including acting as chaperones to other cellular proteins (called client proteins) to facilitate their proper folding and repair, and to aid in the refolding of misfolded client proteins. There are several known families of HSPs, each having its own set of client proteins. Hsp90 is one of the most abundant HSP families, accounting for about 1-2% of proteins in a cell that is not under stress and increasing to about 4-6% in a cell under stress.

Inhibition of Hsp90 results in degradation of its client proteins via the ubiquitin proteasome pathway. Unlike other chaperone proteins, the client proteins of Hsp90 are mostly protein kinases or transcription factors involved in signal transduction, and a number of its client proteins have been shown to be involved in the progression of cancer. Hsp90 has been shown by mutational analysis to be necessary for the survival of normal eukaryotic cells. However, Hsp90 is overexpressed in many tumor types, indicating that it may play a significant role in the survival of cancer cells and that cancer cells may be more sensitive to inhibition of Hsp90 than normal cells. For example, cancer cells typically have a large number of mutated and overexpressed oncoproteins that are dependent on Hsp90 for folding. In addition, because the environment of a tumor is typically hostile due to hypoxia, nutrient deprivation, acidosis, etc., tumor cells may be especially dependent on Hsp90 for survival. Moreover, inhibition of Hsp90 causes simultaneous inhibition of a number of oncoproteins, as well as hormone receptors and transcription factors, making it an attractive target for an anti-cancer agent.

Despite the emergence of various therapies, chemotherapy still plays a significant role in the treatment of cancer. Though toxic, the potency and broad applicability of chemotherapeutic drugs make compelling rationale for using them as payloads in drug delivery systems. Several recent technologies, especially antibody drug conjugates (ADC) have been very successful, however for narrow sets of indications. Although tremendous advances have been made in chemotherapy, currently available therapeutics and therapies remain unsatisfactory and the prognosis for the majority of patients diagnosed with chemotherapeutically treated diseases (*e.g.,* cancer) remains poor. Often, the applicability and/or effectiveness of chemotherapy, as well as other therapies and diagnostics employing potentially toxic moieties, is limited by undesired side effects.

Accordingly, the need exists for improved therapeutic molecules that are designed to selectively release drug to cells.

### SUMMARY OF THE INVENTION

The present invention is based on the Hsp90 inhibitor drug conjugate ("HDC") platform, which utilizes the unique pharmacokinetic property of Hsp90 inhibitors (Hsp90i), where the drug is selectively retained in tumor in high concentrations while clearing from plasma and normal tissues relatively quickly. HDC is a novel and inventive idea, wherein a small molecule HDC inhibitor is used as a delivery vehicle for the delivery of another small molecule *(e.g.,* an anti-cancer agent). An HDC can be created with any small molecule that can offer anchors (functional groups) for conjugation, where the strength of the link between the HSP90 inhibitor and the payload can be manipulated depending on the payload and the intended payload release. The present invention describes HDCs with a linker chemistry that is advantageously designed to be selectively cleaved inside a cell. Accordingly, the HDCs of the present invention release the payload in target cells while maintaining a stable linkage and attenuating drug potency in circulation.

As described herein, the HDC platform has the potential to utilize a number of commonly used small molecule anticancer agents as payloads, expanding their applicability through improved efficacy and toxicity profiles. It also has the potential to resuscitate the development of drugs that were abandoned due to toxicity and lack of efficacy.

Accordingly, in a first aspect, the present invention features an Hsp90 drug conjugate (HDC) comprising an Hsp90 ligand or prodrug thereof, a linker moiety and a payload moiety. In one embodiment, the Hsp90 ligand is an Hsp90 inhibitor.

In one embodiment, the payload moiety is selected from the group consisting of: a therapeutic moiety, a cytotoxic moiety and an imaging moiety. In one embodiment, the therapeutic moiety is a cytotoxic moiety. In another embodiment, the therapeutic moiety is a chemotherapeutic agent. In another embodiment, the therapeutic agent is an immunomodulating agent.

In a further embodiment, the cytotoxic moiety is an auristatin. In a related embodiment, the aurtistatin is Monomethyl auristatin E (MMAE).

In one embodiment, the chemotherapeutic agent is selected from the group consisting of: imids, proteasome inhibitors, PARP inhibitors, cell cycle inhibitors, alkylating agents, anthracyclines, antimetabolites, epigenetic modifiers, hormonal therapy, microtubule stabilizers, platinums and tyrosine kinase inhibitors. In a further embodiment, the chemotherapeutic agent is an ERK kinase inhibitor. In another further embodiment, the chemotherapeutic agent is a MEK inhibitor.

In one embodiment, the cytotoxic moiety is not suitable for administration alone.

In another embodiment, the linker moiety and the payload moiety are covalently attached. In another further embodiment, the Hsp90 ligand and the linker moiety are covalently attached.

In one embodiment, the linker moiety is a cleavable linker. In a related embodiment, the cleavable linker comprises an enzymatically cleavable linker. In another embodiment, the linker is a disulfide linker. In another embodiment, the linker is a dipeptide linker. In a further related embodiment, the dipeptide linker is a valine-citrulline (val-cit) linker or a valine-citrulline p-aminobenzylcarbamate (val-cit-PABC) linker.

In one embodiment, the HDC is able to enter a cell by passive diffusion. In another embodiment, the Hsp90 ligand or prodrug thereof inhibits Hsp90. In another embodiment, the Hsp90 ligand or prodrug thereof does not inhibit Hsp90.

In another aspect, the present invention features a method of treating a disease or disorder in a subject, comprising administering to the subject an effective amount of an HDC, wherein the HDC comprises an Hsp90 ligand or prodrug thereof, a linker moiety and a payload moiety.

In another aspect, the present invention features a method of treating cancer in a subject, comprising administering to the subject an effective amount of an HDC, wherein the HDC comprises an Hsp90 ligand or prodrug thereof, a linker moiety and a payload moiety. In one embodiment, the method further comprises administering an additional anticancer therapy.

In another aspect, the invention features a kit comprising an HDC, wherein the HDC comprises an Hsp90 ligand or prodrug thereof, a linker moiety and a payload moiety, and instructions for use in treating a disease or disorder. In one embodiment, the disease or disorder is cancer.

The present invention is described in further detail by the figures and examples below, which are used only for illustration purposes and are not limiting.

### BRIEF DESCRIPTION OF THE DRAWINGS

**Figure 1** shows examples of the structural matrix of the HDCs of the present invention.
**Figure 2** shows exemplary HDCs of the present invention. Disulfide linkers are shown in Figure 2(a) SDC-TRAP-0535 and Figure 2(b) SDC-TRAP-0522. Dipeptide linkers are shown in Figure 2(c) SDC-TRAP-0504 and Figure 2(d).

### DETAILED DESCRIPTION OF THE INVENTION

### Definitions

In order that the present invention may be more readily understood, certain terms are first defined. In addition, it should be noted that whenever a value or range of values of a parameter are recited, it is intended that values and ranges intermediate to the recited values are also intended to be part of this invention. Unless defined otherwise, all technical and scientific terms used herein have the same meaning as commonly understood to one of ordinary skill in the art to which this invention belongs. It is also to be understood that the terminology employed is for the purpose of describing particular embodiments, and is not intended to be limiting.

The articles "a", "an" and "the" are used herein to refer to one or to more than one (*i.e*., to at least one) of the grammatical object of the article unless otherwise clearly indicated by contrast. By way of example, "an element" means one element or more than one element.

The term "including" is used herein to mean, and is used interchangeably with, the phrase "including but not limited to."

The term "or" is used herein to mean, and is used interchangeably with, the term "and/or," unless context clearly indicates otherwise.

The term "such as" is used herein to mean, and is used interchangeably, with the phrase "such as but not limited to."

Unless specifically stated or obvious from context, as used herein, the term "about" is understood as within a range of normal tolerance in the art, for example within 2 standard deviations of the mean. About can be understood as within 10%, 9%, 8%, 7%, 6%, 5%, 4%, 3%, 2%, 1%, 0.5%, 0.1 %, 0.05%, or 0.01 % of the stated value. Unless otherwise clear from context, all numerical values provided herein can be modified by the term about.

Ranges provided herein are understood to be shorthand for all of the values within the range. For example, a range of 1 to 50 is understood to include any number, combination of numbers, or sub-range from the group consisting 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50.

The recitation of a listing of chemical group(s) in any definition of a variable herein includes definitions of that variable as any single group or combination of listed groups. The recitation of an embodiment for a variable or aspect herein includes that embodiment as any single embodiment or in combination with any other embodiments or portions thereof.

Any compositions or methods provided herein can be combined with one or more of any of the other compositions and methods provided herein.

The terms "administer," "administering" or "administration" include any method of delivery of a pharmaceutical composition or agent into a subject's system or to a particular region in or on a subject. In certain embodiments of the invention, an agent is administered intravenously, intramuscularly, subcutaneously, intradermally, intranasally, orally, transcutaneously, or mucosally. In a preferred embodiment, an agent is administered intravenously. Administering an agent can be performed by a number of people working in concert. Administering an agent includes, for example, prescribing an agent to be administered to a subject and/or providing instructions, directly or through another, to take a specific agent, either by self-delivery, *e.g.,* as by oral delivery, subcutaneous delivery, intravenous delivery through a central line, etc.; or for delivery by a trained professional, *e.g*., intravenous delivery, intramuscular delivery, intratumoral delivery, etc.

The term "alkyl" by itself or as part of another term refers to a substituted or unsubstituted a straight chain or branched, saturated or unsaturated hydrocarbon having the indicated number of carbon atoms (*e.g.*, "-C₁-C₈ alkyl" or "-C₁-C₁₀ alkyl refer to an alkyl group having from 1 to 8 or 1 to 10 carbon atoms, respectively). When the number of carbon atoms is not indicated, the alkyl group has from 1 to 8 carbon atoms. Representative straight chain "-C₁-C₈ alkyl" groups include, but are not limited to, -methyl, -ethyl, -n-propyl, -n-butyl, -n-pentyl, - n-hexyl, -n- heptyl and -n-octyl; while branched -C₁-C₈ alkyls include, but are not limited to, - isopropyl, -sec-butyl, -isobutyl, -tert-buty\, -isopentyl, and -2-methylbutyl; unsaturated -C₂-C₈ alkyls include, but are not limited to, -vinyl, -allyl, -1-butenyl, -2-butenyl, -isobutylenyl, -1-pentenyl, -2-pentenyl, -3 -methyl- 1-butenyl, -2-methyl-2-butenyls -2,3-dimethyl-2-butenyl, -1-hexyl, 2-hexyl, -3-hexyl, -acetylenyl, -propynyl, -1-butynyl, -2-butynyl, -1-pentynyl, -2-pentynyl and -3 -methyl -1 butynyl. In some embodiments, an alkyl group is unsubstituted. In other embodiments, an alkyl group is substituted with one or more groups. Preferred substitutents include: -0-(C₁-Cg alkyl), aryl, -C(O)R', -OC(O)R', -C(O)OR', -C(O)NH₂, -OH, -C(O)NHR', - C(O)N(R')₂, -NHC(O)R', -SO₃R', -S(O)₂R' -S(O)R', -SR', -halogen, -N₃, -NH₂, -NH(R'), -N(R')₂ and -CN; where each R' is independently selected the group consisting of H, unsubstituted C₁-Cg alkyl and aryl. Particularly preferred substituents include: -OH, -SCH₃, -CONH₂, -COOH, - NHC(=NH)NH₂, -NH₂, -NHCOCH₃, -NHCHO, and -NHCONH₂.

The terms "cancer" or "tumor" are well known in the art and refer to the presence, *e.g.,* in a subject, of cells possessing characteristics typical of cancer-causing cells, such as uncontrolled proliferation, immortality, metastatic potential, rapid growth and proliferation rate, decreased cell death/apoptosis, and certain characteristic morphological features. Cancer cells are often in the form of a solid tumor. However, cancer also includes non-solid tumors, *e.g.,* blood tumors, *e.g.,* leukemia, wherein the cancer cells are derived from bone marrow. As used herein, the term "cancer" includes pre-malignant as well as malignant cancers. Cancers include, but are not limited to, acoustic neuroma, acute leukemia, acute lymphocytic leukemia, acute myelocytic leukemia (monocytic, myeloblastic, adenocarcinoma, angiosarcoma, astrocytoma, myelomonocytic and promyelocytic), acute T-cell leukemia, basal cell carcinoma, bile duct carcinoma, bladder cancer, brain cancer, breast cancer, bronchogenic carcinoma, cervical cancer, chondrosarcoma, chordoma, choriocarcinoma, chronic leukemia, chronic lymphocytic leukemia, chronic myelocytic (granulocytic) leukemia, chronic myelogenous leukemia, colon cancer, colorectal cancer, craniopharyngioma, cystadenocarcinoma, diffuse large B-cell lymphoma, Burkitt's lymphoma, dysproliferative changes (dysplasias and metaplasias), embryonal carcinoma, endometrial cancer, endotheliosarcoma, ependymoma, epithelial carcinoma, erythroleukemia, esophageal cancer, estrogen-receptor positive breast cancer, essential thrombocythemia, Ewing's tumor, fibrosarcoma, follicular lymphoma, germ cell testicular cancer, glioma, heavy chain disease, hemangioblastoma, hepatoma, hepatocellular cancer, hormone insensitive prostate cancer, leiomyosarcoma, liposarcoma, lung cancer, lymphagioendotheliosarcoma, lymphangiosarcoma, lymphoblastic leukemia, lymphoma (Hodgkin's and non-Hodgkin's), malignancies and hyperproliferative disorders of the bladder, breast, colon, lung, ovaries, pancreas, prostate, skin, and uterus, lymphoid malignancies of T-cell or B-cell origin, leukemia, lymphoma, medullary carcinoma, medulloblastoma, melanoma, meningioma, mesothelioma, multiple myeloma, myelogenous leukemia, myeloma, myxosarcoma, neuroblastoma, non-small cell lung cancer, oligodendroglioma, oral cancer, osteogenic sarcoma, ovarian cancer, pancreatic cancer, papillary adenocarcinomas, papillary carcinoma, pinealoma, polycythemia vera, prostate cancer, rectal cancer, renal cell carcinoma, retinoblastoma, rhabdomyosarcoma, sarcoma, sebaceous gland carcinoma, seminoma, skin cancer, small cell lung carcinoma, solid tumors (carcinomas and sarcomas), small cell lung cancer, stomach cancer, squamous cell carcinoma, synovioma, sweat gland carcinoma, thyroid cancer, Waldenstrom's macroglobulinemia, testicular tumors, uterine cancer, and Wilms' tumor. Other cancers include primary cancer, metastatic cancer, oropharyngeal cancer, hypopharyngeal cancer, liver cancer, gall bladder cancer, bile duct cancer, small intestine cancer, urinary tract cancer, kidney cancer, urothelium cancer, female genital tract cancer, uterine cancer, gestational trophoblastic disease, male genital tract cancer, seminal vesicle cancer, testicular cancer, germ cell tumors, endocrine gland tumors, thyroid cancer, adrenal cancer, pituitary gland cancer, hemangioma, sarcoma arising from bone and soft tissues, Kaposi's sarcoma, nerve cancer, ocular cancer, meningial cancer, glioblastomas, neuromas, neuroblastomas, Schwannomas, solid tumors arising from hematopoietic malignancies such as leukemias, metastatic melanoma, recurrent or persistent ovarian epithelial cancer, fallopian tube cancer, primary peritoneal cancer, gastrointestinal stromal tumors, colorectal cancer, gastric cancer, melanoma, glioblastoma multiforme, non-squamous non-small-cell lung cancer, malignant glioma, epithelial ovarian cancer, primary peritoneal serous cancer, metastatic liver cancer, neuroendocrine carcinoma, refractory malignancy, triple negative breast cancer, HER2- amplified breast cancer, nasopharageal cancer, oral cancer, biliary tract, hepatocellular carcinoma, squamous cell carcinomas of the head and neck (SCCHN), non-medullary thyroid carcinoma, recurrent glioblastoma multiforme, neurofibromatosis type 1, CNS cancer, liposarcoma, leiomyosarcoma, salivary gland cancer, mucosal melanoma, acral/ lentiginous melanoma, paraganglioma, pheochromocytoma, advanced metastatic cancer, solid tumor, triple negative breast cancer, colorectal cancer, sarcoma, melanoma, renal carcinoma, endometrial cancer, thyroid cancer, rhabdomysarcoma, multiple myeloma, ovarian cancer, glioblastoma, gastrointestinal stromal tumor, mantle cell lymphoma, and refractory malignancy. A "solid tumor," as used herein, is understood as any pathogenic tumor that can be palpated or detected using imaging methods as an abnormal growth having three dimensions. A solid tumor is differentiated from a blood tumor such as leukemia. However, cells of a blood tumor are derived from bone marrow; therefore, the tissue producing the cancer cells is a solid tissue that can be hypoxic.

As used herein, the term "effective amount" refers to an amount of a compound described herein which is sufficient to reduce or ameliorate the severity, duration, progression, or onset of a disease or disorder, delay onset of a disease or disorder, retard or halt the advancement of a disease or disorder, cause the regression of a disease or disorder, prevent or delay the recurrence, development, onset or progression of a symptom associated with a disease or disorder, or enhance or improve the therapeutic effect(s) of another therapy. The precise amount of compound administered to a subject will depend on the mode of administration, the type and severity of the disease or condition and on the characteristics of the subject, such as general health, age, sex, body weight and tolerance to drugs. For example, for a proliferative disease or disorder, determination of an effective amount will also depend on the degree, severity and type of cell proliferation. The skilled artisan will be able to determine appropriate dosages depending on these and other factors. When co-administered with other therapeutic agents, *e.g*., when co-administered with an anti-cancer agent, an "effective amount" of any additional therapeutic agent(s) will depend on the type of drug used. Suitable dosages are known for approved therapeutic agents and can be adjusted by the skilled artisan according to the condition of the subject, the type of condition(s) being treated and the amount of a compound of the invention being used. In cases where no amount is expressly noted, an effective amount should be assumed. Non-limiting examples of an effective amount of a compound described herein are provided herein below.

As used herein, an "Hsp90 inhibitor," is meant to refer to a therapeutic agent that reduces the activity of Hsp90 either by directly interacting with Hsp90 or by, for example, preventing the formation of the Hsp90/CDC37 complex such that the expression and proper folding of at least one client protein of Hsp90 is inhibited. "Hsp90" includes each member of the family of heat shock proteins having a mass of about 90-kilo Daltons. For example, in humans the highly conserved Hsp90 family includes cytosolic Hsp90^{α} and Hsp90^{β} isoforms, as well as GRP94, which is found in the endoplasmic reticulum, and HSP75/TRAP1, which is found in the mitochondrial matrix.

As used herein, the terms "identify" or "select" refer to a choice in preference to another. In other words, to identify a subject or select a subject is to perform the active step of picking out that particular subject from a group and confirming the identity of the subject by name or other distinguishing feature.

As used herein, the term "in combination" refers to the use of more than one therapeutic agent. The use of the term "in combination" does not restrict the order in which the therapeutic agents are administered to a subject with a disease or disorder, *e.g.,* a proliferative disorder. A first therapeutic agent, such as a compound described herein, can be administered prior to (*e.g.,* 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks before), concomitantly with, or subsequent to (*e.g.,* 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks after) the administration of a second therapeutic agent, such as an anti-cancer agent, to a subject with a disease or disorder, *e.g.,* a proliferative disorder, such as cancer.

The term "linker" or "linking moiety," as used herein refers to a chemical moiety that joins two other moieties (*e.g.,* an Hsp90 inhibitor and an agent). A linker can covalently join an Hsp90 inhibitor and an agent. A linker can include a cleavable linker, for example an enzymatically cleavable linker. A linker can include a disulfide linker, a dipeptide liker, or an alkyl linker.

As used herein, the term "payload" or "payload moiety" refers to any therapeutic, cytotoxic or imaging agent. The term "therapeutic agent" refers to molecule, compound, or fragment thereof that is used for the treatment of a disease or for improving the well-being of an organism or that otherwise exhibit healing power (*e.g.,* pharmaceuticals, drugs, and the like). A therapeutic moiety can be a chemical, or fragment thereof, of natural or synthetic origin used for its specific action against disease, for example cancer. Any agent that exerts a therapeutic effect on cancer cells can be used as the therapeutic agent for conjugation to an Hsp90 inhibitor"". Therapeutic agents used for treating cancer may be called chemotherapeutic agents. As described herein, a therapeutic moiety is preferentially a small molecule. The term "cytotoxic agent" refers to molecule, compound, or fragment thereof that has a toxic or poisonous effect on cells, or that kills cells. Chemotherapy and radiotherapy are forms of cytotoxic therapy. Treating cells with a cytotoxic moiety can produce a variety of results - cells may undergo necrosis, stop actively growing and dividing, or activate a genetic program of controlled cell death (*i.e.,* apoptosis). The term "imaging agent" refers to a molecule, compound, or fragment thereof that facilitates a technique and/or process used to create images or take measurements of a cell, tissue, and/or organism (or parts or functions thereof) for clinical and/or research purposes. An imaging moiety can produce, for example, a signal through emission and/or interaction with electromagnetic, nuclear, and/or mechanical (*e.g*., acoustic as in ultrasound) energy. An imaging moiety can be used, for example, in various radiology, nuclear medicine, endoscopy, thermography, photography, spectroscopy, and microscopy methods.

In exemplary embodiments, the payload moiety is an auristatin. In other exemplary embodiments, the payload moiety is an ERK kinase inhibitor, for example BVD-523. In other exemplary embodiments, the payload moiety is a MEK inhibitor, for example TAK-733.

As used herein, the term "auristatin" refers to a family of antimitotic agents. Auristatin derivatives are also included within the definition of the term "auristatin". Examples of auristatins include, but are not limited to, auristatin E (AE), monomethyl auristatin E (MMAE), monomethyl auristatin F (MMAF), and synthetic analogs of dolastatin. Auristatins have been shown to interfere with microtubule dynamics, GTP hydrolysis, and/or nuclear and cellular division, and have anticancer and/or antifungal activity.

As used herein, the term "pharmaceutically acceptable salt" refers to a salt prepared from a compound of formulae (I) or (Ia), and a pharmaceutically acceptable inorganic or organic base. Suitable bases include hydroxides of alkali metals such as sodium, potassium, and lithium; hydroxides of alkaline earth metal such as calcium and magnesium; hydroxides of other metals, such as aluminum and zinc; ammonia, and organic amines, such as unsubstituted or hydroxy-substituted mono-, di-, or trialkylamines; dicyclohexylamine; tributyl amine; pyridine; N-methyl,N-ethylamine; diethylamine; triethylamine; mono-, bis-, or tris-(2-hydroxy-lower alkyl amines), such as mono-, bis-, or tris-(2-hydroxyethyl)amine, 2-hydroxy-tert-butylamine, or tris-(hydroxymethyl)methylamine, N,N,-di-lower alkyl-N-(hydroxy lower alkyl)-amines, such as N,N-dimethyl-N-(2-hydroxyethyl)amine, or tri-(2-hydroxyethyl)amine; N-methyl-D-glucamine; and amino acids such as arginine, lysine, and the like.

A pharmaceutically acceptable carrier may contain inert ingredients which do not unduly inhibit the biological activity of the compound(s) described herein. The pharmaceutically acceptable carriers should be biocompatible, *i.e.,* non-toxic, non-inflammatory, non-immunogenic and devoid of other undesired reactions upon the administration to a subject. Standard pharmaceutical formulation techniques can be employed, such as those described in Remington, J. P., Remington's Pharmaceutical Sciences (Mack Pub. Co., 17th ed., 1985). Suitable pharmaceutical carriers for parenteral administration include, for example, sterile water, physiological saline, bacteriostatic saline (saline containing about 0.9% mg/ml benzyl alcohol), phosphate-buffered saline, Hank's solution, Ringer's-lactate, and the like. Methods for encapsulating compositions, such as in a coating of hard gelatin or cyclodextran, are known in the art. See Baker et al., Controlled Release Of Biological Active Agents, (John Wiley and Sons, 1986).

As used herein, and unless otherwise indicated, the term "prodrug" means a derivative of a compound that can hydrolyze, oxidize, or otherwise react under biological conditions (*in vitro* or *in vivo*) to provide a compound described herein. Prodrugs may become active upon such reaction under biological conditions, or they may have activity in their unreacted forms. Examples of prodrugs contemplated herein include analogs or derivatives of compounds of formulae (I) or (Ia) that comprise biohydrolyzable moieties such as biohydrolyzable amides, biohydrolyzable esters, biohydrolyzable carbamates, biohydrolyzable carbonates, biohydrolyzable ureides and phosphate analogues. Prodrugs can typically be prepared using well-known methods, such as those described by Burger's Medicinal Chemistry And Drug Discovery, (Manfred E. Wolff Ed., 5th ed. (1995)) 172-178, 949-982.

As used herein, the terms "respond" or "response" are understood as having a positive response to treatment with a therapeutic agent, wherein a positive response is understood as having a decrease in at least one sign or symptom of a disease or condition (*e.g*., tumor shrinkage, decrease in tumor burden, inhibition or decrease of metastasis, improving quality of life ("QOL"), delay of time to progression ("TTP"), increase of overall survival ("OS"), etc.), or slowing or stopping of disease progression (*e.g*., halting tumor growth or metastasis, or slowing the rate of tumor growth or metastasis). A response can also include an improvement in quality of life, or an increase in survival time or progression free survival.

As used herein, the terms "subject", "patient" and "mammal" are used interchangeably. The terms "subject" and "patient" refer to an animal (*e.g*., a bird such as a chicken, quail or turkey, or a mammal), preferably a mammal including a non-primate (*e.g*., a cow, pig, horse, sheep, rabbit, guinea pig, rat, cat, dog, and mouse) and a primate (*e.g*., a monkey, chimpanzee and a human), and more preferably a human. In one embodiment, the subject is a non-human animal such as a farm animal *(e.g.,* a horse, cow, pig or sheep), or a pet *(e.g.,* a dog, cat, guinea pig or rabbit). In another embodiment, the subject is a human.

As used herein, the term "synergistic" refers to a combination of a compound described herein and another therapeutic agent, which, when taken together, is more effective than the additive effects of the individual therapies. A synergistic effect of a combination of therapies *(e.g.,* a combination of therapeutic agents) permits the use of lower dosages of one or more of the therapeutic agent(s) and/or less frequent administration of the agent(s) to a subject with a disease or disorder, *e.g.,* a proliferative disorder. The ability to utilize lower the dosage of one or more therapeutic agent and/or to administer the therapeutic agent less frequently reduces the toxicity associated with the administration of the agent to a subject without reducing the efficacy of the therapy in the treatment of a disease or disorder. In addition, a synergistic effect can result in improved efficacy of agents in the prevention, management or treatment of a disease or disorder, *e.g.,* a proliferative disorder. Finally, a synergistic effect of a combination of therapies may avoid or reduce adverse or unwanted side effects associated with the use of either therapeutic agent alone.

As used herein, the terms "treat", "treatment" and "treating" refer to the reduction or amelioration of the progression, severity and/or duration of a disease or disorder, delay of the onset of a disease or disorder, or the amelioration of one or more symptoms (preferably, one or more discernible symptoms) of a disease or disorder, resulting from the administration of one or more therapies (*e.g*., one or more therapeutic agents such as a compound of the invention). The terms "treat", "treatment" and "treating" also encompass the reduction of the risk of developing a disease or disorder, and the delay or inhibition of the recurrence of a disease or disorder. In one embodiment, the disease or disorder being treated is cancer. In specific embodiments, the terms "treat", "treatment" and "treating" refer to the amelioration of at least one measurable physical parameter of a disease or disorder, such as growth of a tumor, not necessarily discernible by the patient. In other embodiments the terms "treat", "treatment" and "treating" refer to the inhibition of the progression of a disease or disorder, *e.g.,* a proliferative disorder, either physically by the stabilization of a discernible symptom, physiologically by the stabilization of a physical parameter, or both. In another embodiment, the terms "treat", "treatment" and "treating" of a proliferative disease or disorder refers to the reduction or stabilization of tumor size or cancerous cell count, and/or delay of tumor formation. In another embodiment, the terms "treat", "treating" and "treatment" also encompass the administration of a compound described herein as a prophylactic measure to patients with a predisposition (genetic or environmental) to any disease or disorder described herein.

The recitation of an embodiment for a variable or aspect herein includes that embodiment as any single embodiment or in combination with any other embodiments or portions thereof.

The invention can be understood more fully by reference to the following detailed description and illustrative examples, which are intended to exemplify non-limiting embodiments of the invention.

### Hsp90 Drug Conjugates

The present invention provides Hsp90 drug conjugates (HDCs), as well as HDC compositions, kits, and methods of use thereof. HDCs include an Hsp90 ligand or prodrug thereof, a linker moiety and a payload moiety. HDCs are useful in a variety of therapeutic, imaging, diagnostic, and/or research applications. In one illustrative example of cancer therapy, an HDC can be a pharmaceutical conjugate of an Hsp90 ligand or inhibitor associated with a linker moiety and a chemotherapeutic agent. The HDC drug conjugates described by the present invention provide selective cleavage inside a target cell, prefereably a tumor cell.

In terms of function, HDC molecules have a targeting functionality and effector functionality (*e.g*., therapeutic, imaging, diagnostic). These functions are provided by corresponding chemical moieties that can be different (or, in some cases, the same). HDCs can include any one or more Hsp90 ligands conjugated to any one or more payload moieties. In some embodiments, a composition or method can include a combination of two or more Hsp90 ligands and/or two or more payload moieties (*e.g*., a combination therapy and/or multi target therapy) embodied in one or more different types of HDCs.

In various embodiments, HDCs are further characterized by their ability to passively diffuse and/or be actively transported into a target cell of interest. The diffusion and/or transport properties of the HDC can be derived, at least in part, from ionic, polar, and/or hydrophobic properties of the HDC. In preferred embodiments, the HDC enters cells primarily by passive diffusion.

The diffusion and/or transport properties of the HDC can be derived, at least in part, from the molecular weight of the Hsp90 ligand or prodrug, the linker moiety and the payload moiety. HDCs are desirably small, such as in comparison to antibody-drug conjugates ("ADCs"). For example, the molecular weight of an HDC can be less than about 1600, 1500, 1400, 1300, 1200, 1100, 1000, 900, 800, 700, 600, 500, or 400 Daltons.

Delivery of a payload moiety by an HDC can result in greater potency compared to administering an untargeted drug comprising the same payload moiety, for example, because the HDC can be localized at a desired target for an extended period of time through the association of an Hsp90 ligand and its target. Such localization can cause a payload moiety to be active and/or released in a target cell and/or tissue over an extended period of time. This resonance time can be selected through deliberate design of a linker moiety. In contrast, administration of the drug by itself *in vivo* can be more apt to have a shorter resonance time in a given target cell and/or tissue - if it traverses into the cell at all - due to the lack of an "anchor" within the cell.

Drugs that develop resistance due to efflux mechanism can be efficiently used as payloads in HDC, utilizing the unique intracellular retention phenomenon. Moreover, the range of potency of payload is very broad as extremely potent or mildly potent payloads can be used.

HDCs, in part because they comprise an Hsp90 ligand or prodrug thereof, and are relatively small in size, can be efficiently taken up or internalized by a target cell. Conversely, uptake or internalization is relatively inefficient for antibody drug conjugates (ADCs), which must deal with limited antigen expression and relatively inefficient internalization mechanisms for the antibody portion of the molecule.

HDCs in accordance with the present invention can represent a significant advance over the state of the art in targeted drugs. HDCs have broad application in many therapeutic, imaging, and diagnostic application. As discussed above, HDCs are advantageously small in comparison to ADCs, enabling better penetration of solid tumors and more rapid clearance from normal tissues (*e.g*., reduced toxicity). HDCs provide for delivery of therapeutic agents that, because of their toxicity, cannot be used as a drug itself. The design of HDCs (*e.g.,* a structure-property relationship) can be established using methods and rationales within the grasp of those of ordinary skill in the art, and companion imaging diagnostics for targeted therapies may also easily be provided, in view of the simpler chemistry involved.

The HDCs described by the present invention can be used such that an Hsp90 inhibitor is used as a delivery vehicle where the antitumor activity is achieved by payload only. In another embodiment, the antitumor effect is obtained by the action of both Hsp90 inhibitor and the payload.

One illustrative embodiment involves a conjugate of an Hsp90 ligand or prodrug thereof, a linker moiety and an ERK kinase inhibitor, and in particular BVD-523 (ulixertinib).

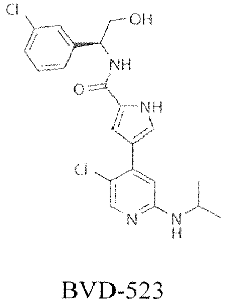

BVD-523 is an orally available inhibitor of extracellular signal-regulated kinase (ERK) 1 and 2, with potential antineoplastic activity. BVD-523 inhibits both ERK 1 and 2, thereby preventing the activation of ERK-mediated signal transduction pathways. This results in the inhibition of ERK-dependent tumor cell proliferation and survival. The mitogen-activated protein kinase (MAPK)/ERK pathway is often upregulated in a variety of tumor cell types and plays a key role in tumor cell proliferation, differentiation and survival.

Another illustrative embodiment involves a conjugate of an Hsp90 ligand or prodrug thereof, a linker moiety and an MEK kinase inhibitor, and in particular TAK-733.

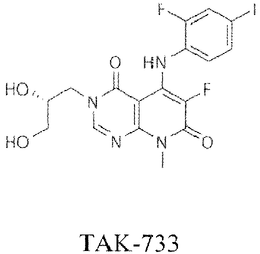

MEK inhibitor TAK-733 selectively binds to and inhibits the activity of MEK1/2, preventing the activation of MEK1/2-dependent effector proteins and transcription factors, which may result in the inhibition of growth factor-mediated cell signaling and tumor cell proliferation. MEK1/2 (MAP2K1/K2) are dual-specificity threonine/tyrosine kinases that play key roles in the activation of the RAS/RAF/MEK/ERK pathway and are often upregulated in a variety of tumor cell types.

Another illustrative embodiment involves a conjugate of an Hsp90 ligand or prodrug thereof, a linker moiety and an auristatin. Auristatins represent a group of dolastatin analogs that have generally been shown to possess anticancer activity by interfering with microtubule dynamics and GTP hydrolysis, thereby inhibiting cellular division. For example, Auristatin E (described in U.S. Patent No. 5,635,483, incorporated by reference herein) is a synthetic analogue of the marine natural product dolastatin 10, a compound that inhibits tubulin polymerization by binding to the same site on tubulin as the anticancer drug vincristine (G. R. Pettit, Prog. Chem. Org. Nat. Prod, 70: 1-79 (1997)). Dolastatin 10, auristatin PE, and auristatin E are linear peptides having four amino acids, three of which are unique to the dolastatin class of compounds. Exemplary embodiments of the auristatin subclass of mitotic inhibitors include, but are not limited to, monomethyl auristatin D (MMAD or auristatin D derivative), monomethyl auristatin E (MMAE or auristatin E derivative), monomethyl auristatin F (MMAF or auristatin F derivative), auristatin F phenylenediamine (AFP), auristatin EB (AEB), auristatin EFP (AEFP), and 5-benzoylvaleric acid-AE ester (AEVB). The synthesis and structure of auristatin derivatives are described in U.S. Patent Application Publication Nos. 2003-0083263, 2005-0238649 and 2005-0009751; International Patent Publication No. WO 04/010957, International Patent Publication No. WO 02/088172, and U.S. Pat. Nos. 6,323,315; 6,239,104; 6,034,065; 5,780,588; 5,665,860; 5,663,149; 5,635,483; 5,599,902; 5,554,725; 5,530,097; 5,521,284; 5,504,191; 5,410,024; 5,138,036; 5,076,973; 4,986,988; 4,978,744; 4,879,278; 4,816,444; and 4,486,414, each of which is incorporated by reference herein.

An exemplary embodiment involves a conjugate of an Hsp90 ligand or prodrug thereof, a linker moiety and an auristatin MMAE (mono-methyl auristatin E). The structure of MMAE is provided below.

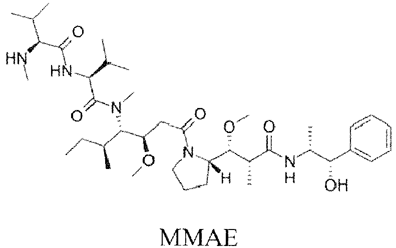

Monomethyl auristatin E (MMAE, vedotin) inhibits cell division by blocking the polymerisation of tubulin. Because of its super toxicity, it also cannot be used as a drug itself. In recent cancer therapy developments, it is linked to a monoclonal antibody (mAb) that recognizes a specific marker expression in cancer cells and directs MMAE to the cancer cells. In one embodiment, the linker linking MMAE to the anti-EGFR antibody is stable in extracellular fluid (*i.e.,* the medium or environment that is external to cells), but is cleaved by cathepsin once the ADC has bound to the specific cancer cell antigen and entered the cancer cell, thus releasing the toxic MMAE and activating the potent anti-mitotic mechanism.

Monomethyl auristatin F (MMAF) is a synthetic antineoplastic agent. MMAF is an antimitotic agent which inhibits cell division by blocking the polymerisation of tubulin The structure of MMAF is shown below:

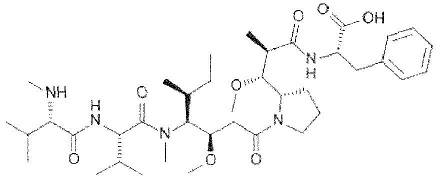

Advantageously, the HDCs of the present invention are designed to be selectively cleaved within the tumor cell.

In one embodiment, HDCs have favorable safety profiles, for example, when compared to, for example, the payload moiety alone.

A number of exemplary HDC molecules are set forth in the examples. Specifically a number of HDC molecules are described and used to demonstrate the efficacy of HDC molecules.

### Hsp90 ligands, or prodrugs thereof

The present invention can exploit the selectively high presence of a molecular target in locations of high physiological activity (*e.g*., Hsp90 in oncological processes). For example, where a drug target is an intracellular drug target, a corresponding molecular target (*e.g*., Hsp90) can be present in the cell. Likewise, where a drug target is an extracellular drug target, a corresponding molecular target *(e.g.,* Hsp90) can be extracellular, proximal, or associated with the extracellular cell membrane of the target cell or tissue.

In various illustrative embodiments where a molecular target of a Hsp90 ligand or prodrug thereof is Hsp90, the Hsp90 ligand can be, for example, a triazole/resorcinol-based compound that binds Hsp90, or a resorcinol amide-based compound that binds Hsp90, *e.g.,* ganetespib, AUY-922 or AT-13387. In another embodiment, the Hsp90 ligand or prodrug thereof may advantageously be an Hsp90-binding compound of formula (I): 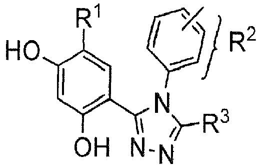 wherein
R¹ may be alkyl, aryl, halide, carboxamide or sulfonamide; R² may be alkyl, cycloalkyl, aryl or heteroaryl, wherein when R² is a 6 membered aryl or heteroaryl, R² is substituted at the 3- and 4-positions relative to the connection point on the triazole ring, through which a linker L is attached; and R³ may be SH, OH, -CONHR⁴, aryl or heteroaryl, wherein when R³ is a 6 membered aryl or heteroaryl, R³ is substituted at the 3 or 4 position.

In another embodiment, the Hsp90 ligand or prodrug thereof may advantageously be an Hsp90-binding compound of formula (II): 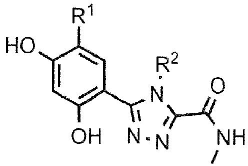 wherein
R¹ may be alkyl, aryl, halo, carboxamido, sulfonamido; and R² may be optionally substituted alkyl, cycloalkyl, aryl or heteroaryl. Examples of such compounds include 5-(2,4-dihydroxy-5-isopropylphenyl)-N-(2-morpholinoethyl)- 4-(4-(morpholinomethyl)phenyl)-4H-1,2,4-triazole-3-carboxamide and 5-(2,4-dihydroxy-5-isopropylphenyl)-4-(4-(4-methylpiperazin-1-yl)phenyl)-N-(2.2,2-trifluoroethyl)-4H-1,2,4-triazole-3-carboxamide.

In another embodiment, the Hsp90 ligand or prodrug thereof may advantageously be an Hsp90-binding compound of formula (III): 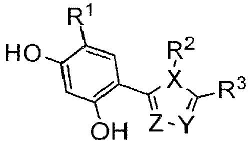 wherein
X, Y, and Z may independently be CH, N, O or S (with appropriate substitutions and satisfying the valency of the corresponding atoms and aromaticity of the ring); R¹ may be alkyl, aryl, halide, carboxamido or sulfonamido; R² may be substituted alkyl, cycloalkyl, aryl or heteroaryl, where a linker L is connected directly or to the extended substitutions on these rings; R³ may be SH, OH, NR⁴R⁵ AND -CONHR⁶, to which an effector moiety may be connected; R⁴ and R⁵ may independently be H, alkyl, aryl, or heteroaryl; and R⁶ may be alkyl, aryl, or heteroaryl, having a minimum of one functional group to which an effector moiety may be connected. Examples of such compounds include AUY-922:

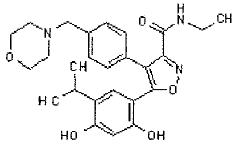

In another embodiment, the Hsp90 ligand or prodrug thereof may advantageously be an Hsp90-binding compound of formula (IV): 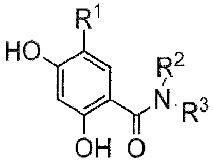 wherein
R¹ may be alkyl, aryl, halo, carboxamido or sulfonamido; R² and R³ are independently C₁-C₅ hydrocarbyl groups optionally substituted with one or more of hydroxy, halogen, C₁-C₂ alkoxy, amino, mono- and di-C₁-C₂ alkylamino; 5- to 12- membered aryl or heteroaryl groups; or, R² and R³, taken together with the nitrogen atom to which they are attached, form a 4- to 8-membered monocyclic heterocyclic group, of which up to 5 ring members are selected from O, N and S. Examples of such compounds include AT-13387:

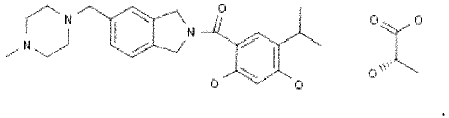

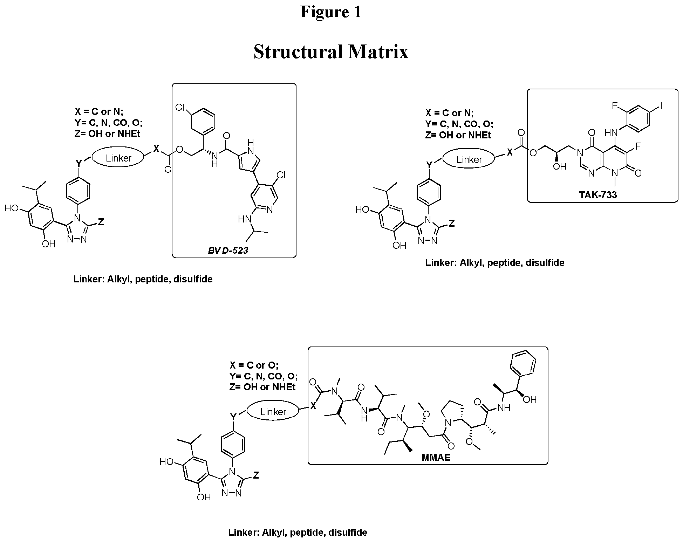

In certain embodiments, to enhance the bioavailability or delivery of the pharmaceutical conjugate, the binding moiety may be a prodrug of the Hsp90-binding compound. Figure 1 shows how the illustrated Hsp90-targeting moiety may be suitably modified at one or more positions to enhance the physical, pharmacokinetic or pharmacodynamic properties of the conjugate.

### Payload Moieties

A payload moiety can be any therapeutic, cytotoxic or imaging agent that can be conjugated to an Hsp90 ligand or prodrug thereof and, in a thus conjugated state, delivered to a molecular target of the Hsp90 ligand. As described in greater detail below, a payload moiety can comprise a region that can be modified and/or participate in covalent linkage to a linker moiety. A payload moiety can be a pharmaceutical molecule or a derivative thereof, which essentially retains activity while conjugated to a linker. It will be appreciated that drugs with otherwise good and desirable activity can prove challenging to administer conventionally (*e.g*., due to poor bioavailability or undesirable side-effects *in vivo* prior to reaching their target) - such drugs can be "reclaimed" for use as effector moieties in the HDCs of the present invention.

Examples of payload moieties include, but are not limited to a therapeutic moiety, a cytotoxic moiety and an imaging moiety.

As described above, in certain preferred embodiments, the payload moiety is an auristatin, for example MMAE. In other preferred embodiments, the payload moiety is an ERK inhibitor, for example BVD-523. In other preferred embodiments, the payload moiety is a MEK inhibitor, for example TAK-733.

### Linker Moieties

Hsp90 ligands, or prodrugs thereof, and payload moieties of the present invention can be conjugated, for example, through a linker moiety L, where L may be either a bond or a linking group. A linker moiety can provide a covalent attachment between a binding moiety and effector moiety. A linker moiety can be inert, for example, with respect to the targeting of a binding moiety and biological activity of a payload moiety.

Appropriate linker moieties can be identified using the affinity, specificity, and/or selectivity assays described herein. Linker moieties can be selected based on size, for example, to provide an HDC with size characteristics as described above. In various embodiments, a linker moiety can be selected, or derived from, known chemical linkers. Linker moieties can comprise a spacer group terminated at either end with a reactive functionality capable of covalently bonding to the drug or ligand moieties. Spacer groups of interest include aliphatic and unsaturated hydrocarbon chains, spacers containing heteroatoms such as oxygen (ethers such as polyethylene glycol) or nitrogen (polyamines), peptides, carbohydrates, cyclic or acyclic systems that may possibly contain heteroatoms. Spacer groups may also be comprised of ligands that bind to metals such that the presence of a metal ion coordinates two or more ligands to form a complex. Specific spacer elements include: 1,4-diaminohexane, xylylenediamine, terephthalic acid, 3,6-dioxaoctanedioic acid, ethylenediamine-N,N-diacetic acid, 1,1'-ethylenebis(5-oxo-3-pyrrolidinecarboxylic acid), 4,4'-ethylenedipiperidine. Potential reactive functionalities include nucleophilic functional groups (amines, alcohols, thiols, hydrazides), electrophilic functional groups (aldehydes, esters, vinyl ketones, epoxides, isocyanates, maleimides), functional groups capable of cycloaddition reactions, forming disulfide bonds, or binding to metals. Specific examples include primary and secondary amines, hydroxamic acids, N-hydroxysuccinimidyl esters, N-hydroxysuccinimidyl carbonates, oxycarbonylimidazoles, nitrophenylesters, trifluoroethyl esters, glycidyl ethers, vinylsulfones, and maleimides. Specific linker moieties that may find use in the HDCs include disulfides and stable thioether moieties.

In some embodiments, the linker moiety of an HDC can be advantageous when compared to the limited linking chemistry of antibody-drug conjugates (ADC). For example, unlike ADCs that are limited by the need to maintain the structure and/or stability of an antibody, HDCs can use a wider range of linking chemistries and/or solvents (*e.g.,* that can alter, distort, or denature an antibody).

In various embodiments, a linker moiety is cleavable, for example enzymatically cleavable. A cleavable linker can be used to release a payload moiety inside a target cell after the HDC is internalized. The susceptibility of a linker moiety to cleavage can be used to control delivery of a payload molecule. For example, a linker moiety can be selected to provide extended or prolonged release of a payload moiety in a target cell over time *(e.g.,* a carbamate linking moiety may be subject to enzymatic cleavage by a carboxylesterase via the same cellular process used to cleave other carbamate prodrugs like capecitabine or irinotecan). In these, and various other embodiments, a linker moiety can exhibit sufficient stability to ensure good target specificity and low systemic toxicity, but not so much stability that it results in lowering the potency and efficacy of the HDC.

In preferred embodiments, the linker is selectively cleaved inside the target cell, preferably a tumor cell. For example, the HDCs of the present invention include linkers that are designed to release the payload (*e.g*., active drug) upon proteolytic cleavage within the lysosomes of target cells, for example tumor cells.

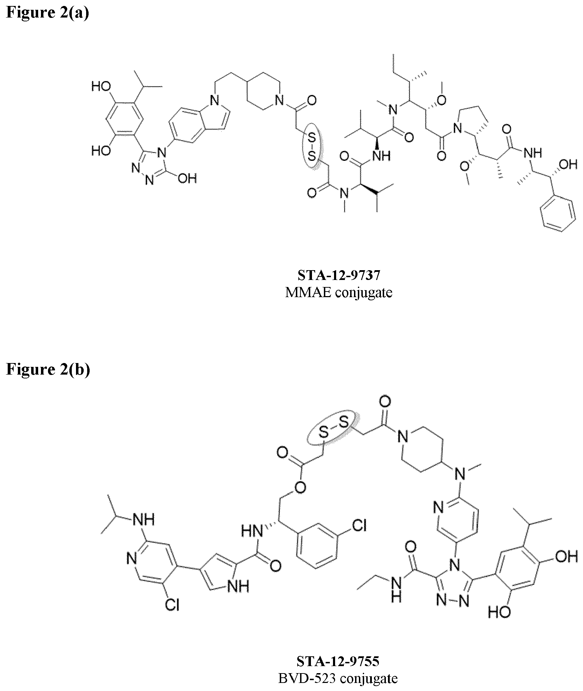

According to preferred embodiments of the present invention, the linker is a disulfide linker. Figure 2(a) and Figure 2(b) show exemplary HDCs with disulfide linkers.

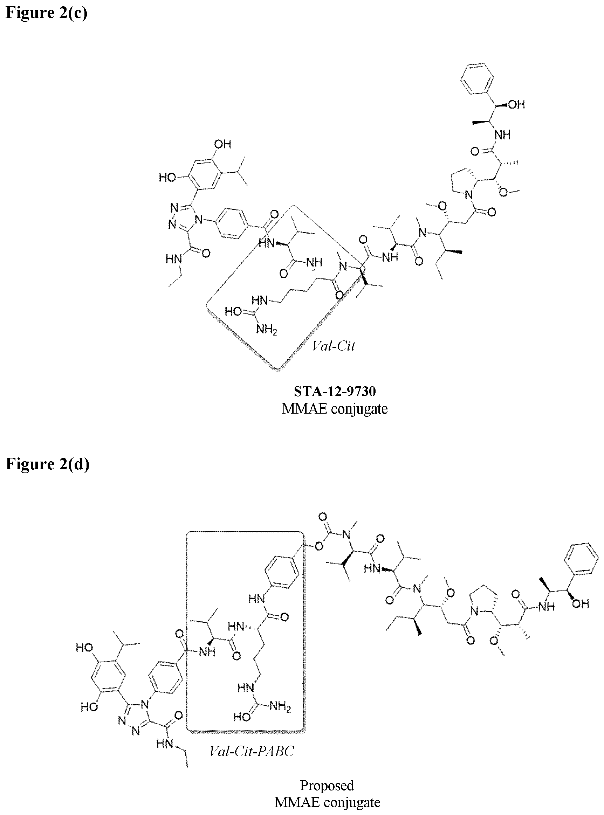

According to preferred embodiments of the present invention, the linker is a dipeptide linker. Figure 2(c) and Figure 2(d) show exemplary HDCs with dipeptide linkers. Exemplary dipeptide linkers include valine-citrulline (val-cit) and valine-citrulline p-aminobenzylcarbamate (val-cit-PABC) linkers. In certain embodiments, the HDC includes a val-cit linker or a val-cit-PABC linker that is cleaved by intracellular proteases such as cathepsin B in the target tumor cell.

### Imaging Moieties, and Diagnostic and Research Applications

In various embodiments, the payload moiety is an imaging moiety - that is, a molecule, compound, or fragment thereof that facilitates a technique and/or process used to create images or take measurements of a cell, tissue, and/or organism (or parts or functions thereof) for clinical and/or research purposes. An imaging moiety can produce, for example, a signal through emission and/or interaction with electromagnetic, nuclear, and/or mechanical (*e.g.,* acoustic as in ultrasound) energy. An imaging moiety can be used, for example, in various radiology, nuclear medicine, endoscopy, thermography, photography, spectroscopy, and microscopy methods.

Imaging studies can be used, for example, in a clinical or research setting to diagnose a subject, select a subject for therapy, select a subject for participation in a clinical trial, monitor the progression of a disease, monitor the effect of therapy, to determine if a subject should discontinue or continue therapy, to determine if a subject has reached a clinical end point, and to determine recurrence of a disease. Imaging studies can be used, for example, to conduct research to identify effective interacting moieties and/or effector moieties and/or combinations thereof, to identify effective dosing and dose scheduling, to identify effective routes of administration, and to identify suitable targets (*e.g.,* diseases susceptible to particular treatment).

### Methods of Making Pharmaceutical Conjugates

The pharmaceutical conjugates, *i.e.,* HDCs, of the invention may be prepared using any convenient methodology. In a rational approach, the pharmaceutical conjugates are constructed from their individual components, binding moiety, in some cases a linker, and effector moiety. The components can be covalently bonded to one another through functional groups, as is known in the art, where such functional groups may be present on the components or introduced onto the components using one or more steps, *e.g.,* oxidation reactions, reduction reactions, cleavage reactions and the like. Functional groups that may be used in covalently bonding the components together to produce the pharmaceutical conjugate include: hydroxy, sulfhydryl, amino, and the like. The particular portion of the different components that are modified to provide for covalent linkage will be chosen so as not to substantially adversely interfere with that components desired binding activity, *e.g*., for the effector moiety, a region that does not affect the target binding activity will be modified, such that a sufficient amount of the desired drug activity is preserved. Where necessary and/or desired, certain moieties on the components may be protected using blocking groups, as is known in the art, *see, e.g.,* Green & Wuts, Protective Groups in Organic Synthesis (John Wiley & Sons) (1991).

Alternatively, the pharmaceutical conjugate can be produced using known combinatorial methods to produce large libraries of potential pharmaceutical conjugates which may then be screened for identification of a bifunctional, molecule with the pharmacokinetic profile. Alternatively, the pharmaceutical conjugate may be produced using medicinal chemistry and known structure-activity relationships for the targeting moiety and the drug. In particular, this approach will provide insight as to where to join the two moieties to the linker.

### Methods of Use, Pharmaceutical Preparations, and Kits

The pharmaceutical conjugates find use in treatment of a host condition, *e.g.,* a disease condition. In these methods, an effective amount of the pharmaceutical conjugate is administered to the host, where "effective amount" means a dosage sufficient to produce the desired result, *e.g.,* an improvement in a disease condition or the symptoms associated therewith. In many embodiments, the amount of drug in the form of the pharmaceutical conjugate that need be administered to the host in order to be an effective amount will vary from that which must be administered in free drug form. The difference in amounts may vary, and in many embodiments may range from two-fold to ten-fold. In certain embodiments, *e.g.,* where the resultant modulated pharmacokinetic property or properties result(s) in enhanced activity as compared to the free drug control, the amount of drug that is an effective amount is less than the amount of corresponding free drug that needs to be administered, where the amount may be two-fold, usually about four-fold and more usually about ten-fold less than the amount of free drug that is administered.

The pharmaceutical conjugate may be administered to the host using any convenient means capable of producing the desired result. Thus, the pharmaceutical conjugate can be incorporated into a variety of formulations for therapeutic administration. More particularly, the pharmaceutical conjugate of the present invention can be formulated into pharmaceutical compositions by combination with appropriate, pharmaceutically acceptable carriers or diluents, and may be formulated into preparations in solid, semi-solid, liquid or gaseous forms, such as tablets, capsules, powders, granules, ointments, solutions, suppositories, injections, inhalants and aerosols. As such, administration of the pharmaceutical conjugate can be achieved in various ways, including oral, buccal, rectal, parenteral, intraperitoneal, intradermal, transdermal, intracheal, etc., administration. In pharmaceutical dosage forms, the pharmaceutical conjugate may be administered alone or in combination with other pharmaceutically active compounds.

The subject methods find use in the treatment of a variety of different disease conditions. In certain embodiments, of particular interest is the use of the subject methods in disease conditions where an active agent or drug having desired activity has been previously identified, but which active agent or drug does not bind to its target with desired affinity and/or specificity. With such active agents or drugs, the subject methods can be used to enhance the binding affinity and/or specificity of the agent for its target.

The specific disease conditions treatable by with the subject HDCs are as varied as the types of drug moieties that can be present in the pharmaceutical conjugate. Thus, disease conditions include cellular proliferative diseases, such as neoplastic diseases, autoimmune diseases, central nervous system or neurodegenerative diseases, cardiovascular diseases, hormonal abnormality diseases, infectious diseases, and the like.

In preferred embodiments, the invention features a method of treating cancer in a subject, comprising administering to the subject an effective amount of an HDC, wherein the HDC comprises an Hsp90 ligand or prodrug thereof, a linker moiety and a payload moiety.

By treatment is meant at least an amelioration of the symptoms associated with the disease condition afflicting the host, where amelioration is used in a broad sense to refer to at least a reduction in the magnitude of a parameter, *e.g*., symptom, associated with the pathological condition being treated, such as inflammation and pain associated therewith. As such, treatment also includes situations where the pathological condition, or at least symptoms associated therewith, are completely inhibited, *e.g.,* prevented from happening, or stopped, *e.g.,* terminated, such that the host no longer suffers from the pathological condition, or at least the symptoms that characterize the pathological condition.

Methods of use of the invention extend beyond strict treatment of a disease. For example, the invention includes uses in a clinical or research setting to diagnose a subject, select a subject for therapy, select a subject for participation in a clinical trial, monitor the progression of a disease, monitor the effect of therapy, to determine if a subject should discontinue or continue therapy, to determine if a subject has reached a clinical end point, and to determine recurrence of a disease.

A variety of hosts are treatable according to the subject methods. Generally such hosts are "mammals" or "mammalian," where these terms are used broadly to describe organisms which are within the class Mammalia, including the orders carnivore (*e.g.*, dogs and cats), rodentia *(e.g.,* mice, guinea pigs, and rats), and primates (*e.g.,* humans, chimpanzees, and monkeys). In many embodiments, the hosts will be humans.

The invention provides kits comprising an HDC, wherein the HDC comprises an Hsp90 ligand or prodrug thereof, a linker moiety and a payload moiety, and instructions for use in treating a disease or disorder.

Kits with unit doses of the pharmaceutical conjugate, usually in oral or injectable doses and often in a storage stable formulation, are provided. In such kits, in addition to the containers containing the unit doses, an informational package insert describing the use and attendant benefits of the drugs in treating pathological condition of interest will be included. Preferred compounds and unit doses are those described herein above.

### EXAMPLES

The following examples, which are briefly summarized and then discussed in turn below, are offered by way of illustration and not by way of limitation.

### Example 1- Hsp90 inhibitor ERK 1/2 kinase inhibitor (BVD-523) conjugates

BVD-523 (ulixertinib) is a targeted cytotoxic designed to inhibit ERK kinase. General procedure for BVD-523 ester conjugation:

### SDC-TRAP-0507

### (S)-2-(4-(5-chloro-2-(isopropylamino)pyridin-4-yl)-1H-pyrrole-2-carboxamido)-2-(3-chlor-ophertyl)ethyl-(1-(4-(3-(2,4-dihydroxy-5-isopropylphenyl)-5-(ethylcarbamoyl)-4H-1,2,4-triazol-4-yl)-2-fluorobenzyl)piperidine-4-carboxamido)cyclopropanecarboxylate

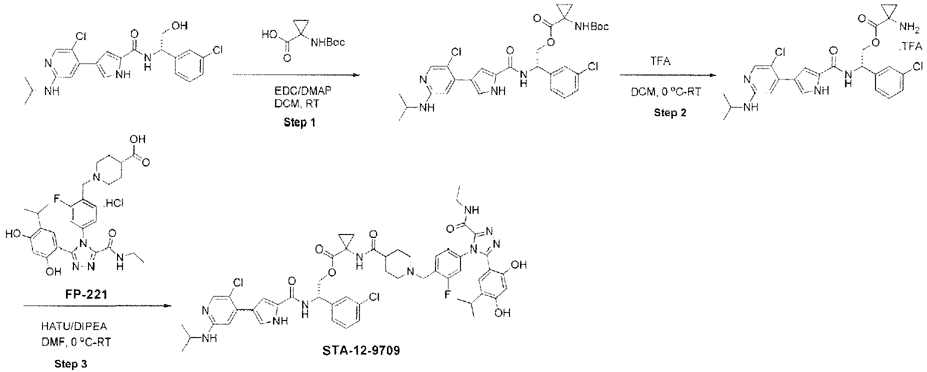

*Step 1:* To a suspension of (S)-4-(5-chloro-2-(isopropylamino)pyridin-4-yl)-N-(1-(3-chlorophenyl)-2-hydroxyethyl)-1H-pyrrole-2-carboxamide (250 mg, 0.576 mmol) and 1-((tert-butoxycarbonyl)amino)cyclopropanecarboxylic acid (145 mg, 0.634 mmol) in dry CH₂Cl₂ (15 mL) was added EDC (121 mg, 0.634 mmol) and DMAP (77 mg, 0.634 mmol). The reaction mixture was stirred at room temperature overnight. The mixture was diluted with DCM and washed with aqueous NH₄Cl solution. The organic layer was dried over Na₂SO₄ and concentrated in vacuum. The crude residue was purified by ISCO using DCM/MeOH (0-10%) as eluent to afford 343 mg (96.6%) of product. ESMS calculated for C₃₀H₃₅Cl₂N₅O₅: 616.54; found: 617 (M+H).

*Step 2:* To a solution of (S)-2-(4-(5-chloro-2-(isopropylamino)pyridin-4-yl)-1H-pyrrole-2-carboxamido)-2-(3-chlorophenyl)ethyl 1-((tert-butoxycarbonyl)amino)cyclopropane carboxylate (43 mg, 0.069 mmol) in dry CH₂Cl₂ (1.6 mL) at 0 °C added TFA (0.4 mL). After 1 h, the ice bath removed and further stirred at room temperature for 1.5 h. The reaction mixture was concentrated, co-evaporated with hexanes several times and vacuum dried. The crude product was obtained as TFA salt and used in the next step without further purification.

*Step 3:* To a mixture of (S)-2-(4-(5-chloro-2-(isopropylamino)pyridin-4-yl)-1H-pyrrole-2-carboxamido)-2-(3-chlorophenyl)ethyl 1-aminocyclopropanecarboxylate (TFA salt) (43 mg, 0.069 mmol) and FP-221 (44 mg, 0.082 mmol) in DMF (3 mL) at 0 °C was added HATU (27 mg, 0.069 mmol) and DIPEA (0.1 mL, 0.56 mmol). After 1 h, the ice bath removed, further stirred at room temperature for 1.5 h. The crude mixture was treated with water, extracted with EtOAc. The organic layer was dried over Na₂SO₄ and concentrated in vacuum. The crude residue was purified by ISCO using DCM/MeOH (0-10%) as eluent to afford 64 mg (70.5%) of SDC-TRAP-0507. ¹H NMR (400 MHz, DMSO-d₆) δ 11.90 (s, 1H). 10.38 (s, 1H), 9.77 (s, 1H), 9.01 (t, J = 6 Hz, 1H), 8.55 (d, J = 8.4 Hz, 1H), 8.39 (s, 1H), 7.97 (s, 1H), 7.48 (s, 1H), 7.41-7.31 (m, 7H), 7.15-7.12 (m, 1H), 6.66 (s, 1H), 6.60 (s, 1H), 6.43 (d, J = 7.6 Hz, 1H), 6.34 (s, 1H), 5.32-5.27 (m, 1H), 4.36-4.23 (m, 2H), 3.96-3.91 (m, 1H), 3.44 (s, 2H), 3.22-3.15 (m, 2H), 2.95-2.90 (m, 1H), 2.75-2.72 (m, 2H), 1.98-1.86 (m, 3H). 1.52-1.44 (m, 4H), 1.29 (s, 2H), 1.12 (d, J = 6.4 Hz, 6H), 1.05 (t, J = 7 Hz, 3H), 0.95-0.92 (m, 2H), 0.85 (d, J = 6.8 Hz, 6H); ESMS calculated (C₅₂H₅₇Cl₂FN₁₀O₇): 1023.98; found: 1024 (M+H).

### SDC-TRAP-0508

### (S)-2-(4-(5-chloro-2-(isopropylamino)pyridin-4-yl)-1H-pyrrole-2-carboxamido)-2-(3-chloraphenyl)ethyl 1-(3-(5-(3-(2,4-dihydroxy-5-isopropylphenyl)-5-hydroxy-4H-1,2,4-triazol-4-yl)-1H-indol-1-yl)propanamido)cyclopropanecarboxylate

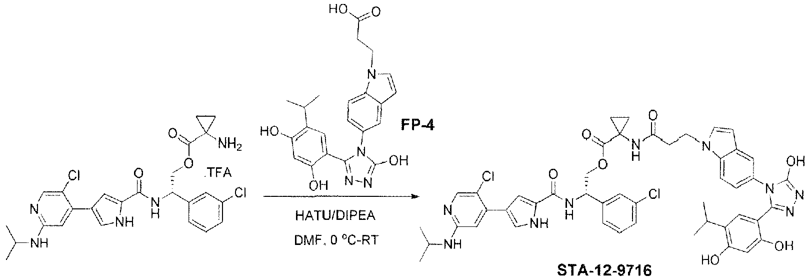

To a mixture of (S)-2-(4-(5-chloro-2-(isopropylamino)pyridin-4-yl)-1H-pyrrole-2-carboxamido)-2-(3-chlorophenyl)ethyl 1-aminocyclopropanecarboxylate (TFA salt) (43 mg, 0.069 mmol) and FP-4 (35 mg, 0.082 mmol) in DMF (3 mL) at 0 °C was added HATU (27 mg, 0.069 mmol) and DIPEA (0.1 mL, 0.56 mmol). After 1 h, the ice bath removed, further stirred at room temperature for 1.5 h. The crude mixture was treated with water, extracted with EtOAc. The organic layer was dried over Na₂SO₄ and concentrated in vacuum. The crude residue was purified by ISCO using DCM/MeOH (0-10%) as eluent to afford 50 mg (79%) of product. ¹H NMR (400 MHz, DMSO-d₆) δ 11.91 (s, 1H), 11.86 (s, 1H), 9.53 (s, 1H), 9.43 (s, 1H), 8.57 (s, 2H), 7.96 (s, 1H), 7.49 (s, 1H), 7.41-7.33 (m, 7H), 7.26-7.25 (m, 1H), 6.90-6.88 (m, 1H). 6.74 (s, 1H), 6.60 (s, 1H), 6.44 (d, J = 8 Hz, 1H), 6.37 (s, 1H), 6.20 (s, 1H), 5.34-5.29 (m, 1H), 4.32-4.23 (m, 4H), 3.98-3.90 (m, 1H), 2.93-2.86 (m, 1H), 2.46-2.43 (m, 2H), 1.25 (broad s, 2H), 1.12 (d, J = 6.4 Hz, 6H), 0.88-0.81 (m, 8H); ESMS calculated (C₄₇H₄₇Cl₂N₉O₇): 920.84; found: 921.8 (M+H).

### SDC-TRAP-0509

### (8)-2-(4-(5-chloro-2-(isopropylamino)pyridin-4-yl)-1H-pyrrole-2-carboxamido)-2-(3-chlorophenyl)ethyl 1-(1-(4-(3-(2,4-dihydroxy-5-isopropylphenyl)-5-hydroxy-4H-1,2, 4-triazol-4-yl)-2-fluorobenzyl)piperidine-4-carboxamido)cyclopropanecarboxylate

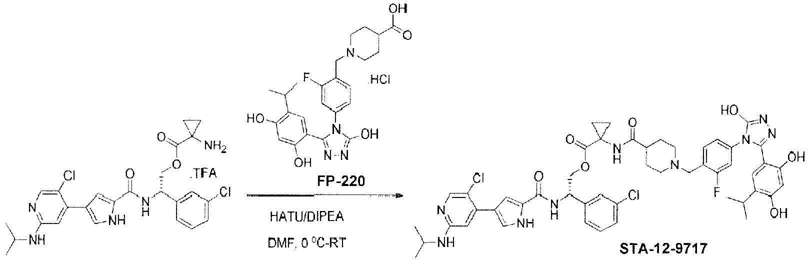

To a mixture of (S)-2-(4-(5-chloro-2-(isopropylamino)pyridin-4-yl)-1H-pyrrole-2-carboxamido)-2-(3-chlorophenyl)ethyl 1-aminocyclopropanecarboxylate (TFA salt) (43 mg, 0.069 mmol) and FP-220 (42 mg, 0.082 mmol) in DMF (3 mL) at 0 °C was added HATU (27 mg, 0.069 mmol) and DIPEA (0.1 mL, 0.56 mmol). After 1 h, the ice bath removed, further stirred at room temperature for 1.5 h. The crude mixture was treated with water, extracted with EtOAc. The organic layer was dried over Na₂SO₄ and concentrated in vacuum. The crude residue was purified by ISCO using DCM/MeOH (0-10%) as eluent to afford 49 mg (74%) of product. ¹H NMR (400 MHz, DMSO-d₆) δ 11.99 (s, 1H), 11.89 (s, 1H), 9.63 (s, 1H), 9.40 (s, 1H), 8.55 (d, J = 8.4 Hz, 1H), 8.36 (s, 1H), 7.96 (s, 1H), 7.47 (s, 1H), 7.37-7.28 (m, 6H), 7.03 (d, J = 8 Hz, 1H), 6.94 (d, J = 8 Hz, 1H), 6.85 (s, 1H), 6.59 (s, 1H), 6.42 (d, J = 8 Hz, 1H), 6.26 (s, 1H), 5.31-5.25 (m, 1H), 4.35-4.21 (m, 2H), 3.96-3.91 (m, 1H), 3.38 (s, 2H), 3.01-2.98 (m, 1H), 2.68 (broad s, 2H), 1.94-1.80 (m, 3H), 1.55-1.40 (m, 4H), 1.30-1.25 (m, 2H), 1.12 (d, J = 6.4 Hz, 6H), 0.99 (d, J = 6.8 Hz, 6H), 0.95-0.93 (m, 2H); ESMS calculated for C₄₉H₅₂Cl₂FN₉O₇: 968.90; found: 969 (M+H).

### SDC-TRAP-0510

### (S)-2-(4-(5-chloro-2-(isopropylamino)pyridin-4-yl)-1H-pyrrole-2-carboxamido)-2-(3-chlorophenyl)ethyl 1-(1-(4-(3-(2,4-dihydroxy-5-isopropylphenyl)-5-(ethylcarbamoyl)-4H-1,2,4-triazol-4-vl)-2-flttorobetizyl)piperidine-4-carbonyl)piperidine-4-carboxylate

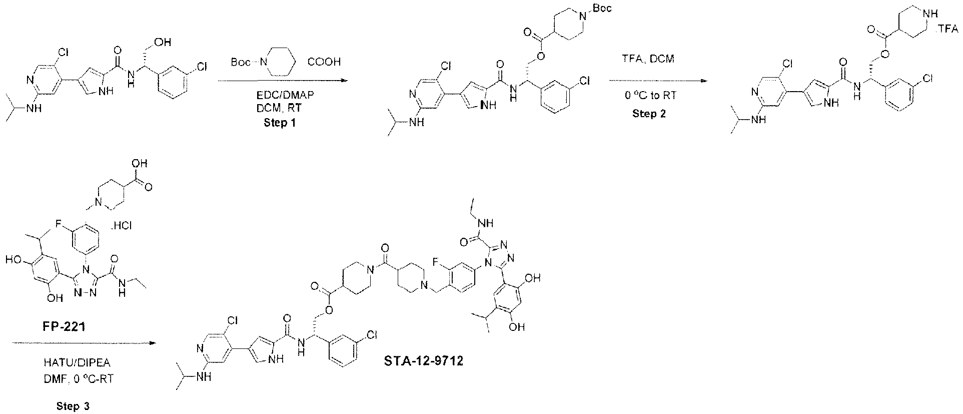

*Step 1:* To a mixture of (S)-4-(5-chloro-2-(isopropylamino)pyridin-4-yl)-N-(1-(3-chlorophenyl)-2-hydroxyethyl)-1H-pyrrole-2-carboxamide (65 mg, 0.15 mmol) and 1-(tert-butoxycarbonyl)piperidine-4-carboxylic acid (38 mg, 0.165 mmol) in dry CH₂Cl₂ (4 mL) was added EDC (32 mg, 0.165 mmol) and DMAP (20 mg, 0.165 mmol). The reaction mixture was stirred at room temperature overnight. The mixture was diluted with DCM and washed with aqueous NH₄Cl solution. The organic layer was dried over Na₂SO₄ and concentrated in vacuum. The crude residue was purified by ISCO using DCM/MeOH (0-10%) as eluent to afford 95 mg (98%) of product. ESMS calculated (C₃₂H₃₉Cl₂N₅O₅): 644.59; found: 645 (M+H).

*Step 2:* To a solution of (S)-1-tert-butyl 4-(2-(4-(5-chloro-2-(isopropylamino)pyridin-4-yl)-1H-pyrrole-2-carboxamido)-2-(3-chlorophenyl)ethyl) piperidine-1,4-dicarboxylate (45 mg, 0.069 mmol) in dry CH₂Cl₂ (1.6 mL) at 0 °C added TFA (0.4 mL). After 1 h, the ice bath removed and further stirred at room temperature for 1.5 h. The reaction mixture was concentrated, co-evaporated with hexanes several times and vacuum dried. The crude product was obtained as TFA salt and used in the next step without further purification.

*Step 3:* To a mixture of (S)-2-(4-(5-chloro-2-(isopropylamino)pyridin-4-yl)-1H-pyrrole-2-carboxamido)-2-(3-chlorophenyl)ethyl piperidine-4-carboxylate (TFA salt) (45 mg, 0.069 mmol) and FP-221 (47 mg, 0.084 mmol) in DMF (3 mL) at 0 °C was added HATU (27 mg, 0.069 mmol) and DIPEA (0.1 mL, 0.56 mmol). After 1 h, the ice bath removed, further stirred at room temperature for 1 h. The crude mixture was treated with water, extracted with EtOAc. The organic layer was dried over Na₂SO₄ and concentrated in vacuum. The crude residue was purified by ISCO using DCM/MeOH (0-10%) as eluent to afford 55 mg (76%) of product. ¹H NMR (400 MHz, DMSO-d₆) δ 11.92 (s, 1H), 10.37 (s, 1H), 9.76 (s, 1H), 9.01 (t, J = 5.6 Hz, 1H), 8.65 (d, J = 8 Hz, 1H), 7.97 (s, 1H), 7.53 (s, 1H), 7.43-7.34 (m, 7H), 7.15 (d, J = 8 Hz, 1H), 6.66 (s, 1H), 6.59 (s, 1H), 6.44 (d, J = 7.8 Hz, 1H), 6.33 (s, 1H), 5.42-5.35 (m, 1H), 4.34-4.33 (m, 2H), 4.05-3.90 (m, 2H), 3.8-3.65 (m, 1H), 3.48 (broad s, 2H), 3.19-3.14 (m, 2H), 3.09-3.06 (m, 1H), 2.97-2.89 (m, 1H), 2.78 (broad s, 3H), 2.62-2.59 (m, 1H), 1.98 (broad s, 2H), 1.80-1.70 (m, 2H), 1.6-1.2 (m, 7H), 1.13 (d, J = 6.4 Hz, 6H), 1.05 (t, J = 7.2 Hz, 3H), 0.86 (d, J = 6.8 Hz, 6H); ESMS calculated (C₅₄H₆₁Cl₂FN₁₀O₇): 1052.03; found: 1053 (M+H).

### SDC-TRAP-0511

### (S)-2-(4-(5-chloro-2-(isopropylamino)pyridin-4-yl)-1H-pyrrole-2-carboxamido)-2-(3-chlorophenyl)ethyl 1-(1-(4-(3-(2,4-dihydroxy-5-isopropylphenyl)-5-hydroxy-4H-1,2,4-triazol-4-yl)-2-fluorobenzyl)piperidine-4-carbonyl)piperidine-4-carboxylate

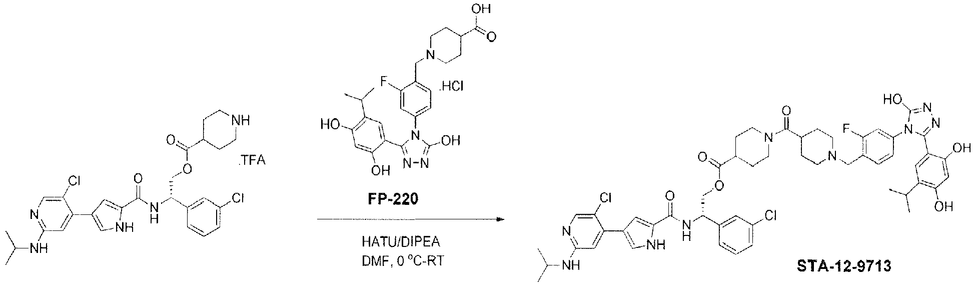

To a mixture of (S)-2-(4-(5-chloro-2-(isopropylamino)pyridin-4-yl)-1H-pyrrole-2-carboxamido)- 2-(3-chlorophenyl)ethyl piperidine-4-carboxylate (TFA salt) (45 mg, 0.069 mmol) and FP-220 (43 mg, 0.084 mmol) in DMF (3 mL) at 0 °C was added HATU (27 mg, 0.069 mmol) and DIPEA (0.1 mL, 0.56 mmol). After 1 h, the ice bath removed, further stirred at room temperature for 1 h. The crude mixture was treated with water, extracted with EtOAc. The organic layer was dried over Na₂SO₄ and concentrated in vacuum. The crude residue was purified by ISCO using DCM/MeOH (0-10%) as eluent to afford 52 mg (76%) of product. ¹H NMR (400 MHz, DMSO-d₆) δ 11.99 (s, 1H), 11.91 (s, 1H), 9.63 (s, 1H), 9.40 (s, 1H), 8.65 (d, J = 8 Hz, 1H), 7.97 (S, 1H), 7.52 (s, 1H), 7.41-7.33 (m, 6H), 7.04 (d, J = 8 Hz, 1H), 6.96 (d, J = 8Hz, 1H), 6.88 (s, 1H), 6.59 (s, 1H), 6.44 (d, J = 7.6 Hz, 1H), 6.26 (s, 1H), 5.39-5.35 (m, 1H), 4.33-4.32 (m, 2H), 4.05-3.94 (m, 2H), 3.71-3.69 (m, 1H), 3.44 (s, 2H), 3.07-2.96 (m, 2H), 2.74 (broad s, 3H), 2.62-2.57 (m, 1H), 1.94 (broad s, 2H), 1.80-1.66 (m, 2H), 1.55-1.23 (m, 7H), 1.13 (d, J = 6.4 Hz, 6H), 0.99 (d, J = 6.78 Hz, 6H); ESMS calculated (C₅₁H₅₆Cl₂FN₉O₇): 996.95; found: 998 (M+H).

### SDC-TRAP-0512

### (S)-2-(4-(5-chloro-2-(isopropylamino)pyridin-4-yl)-1H-pyrrole-2-carboxamido)-2-(3-chlorophenyl)ethyl 2-(1-(1-(4-(3-(2,4-dihydroxy-5-isopropylphenyl)-5-(ethylcarhamoyl)-4H-1,2,4-triazol-4-yl)-2-fluorobenzyl)piperidine-4-carbonyl)piperidin-4-yl)acetate

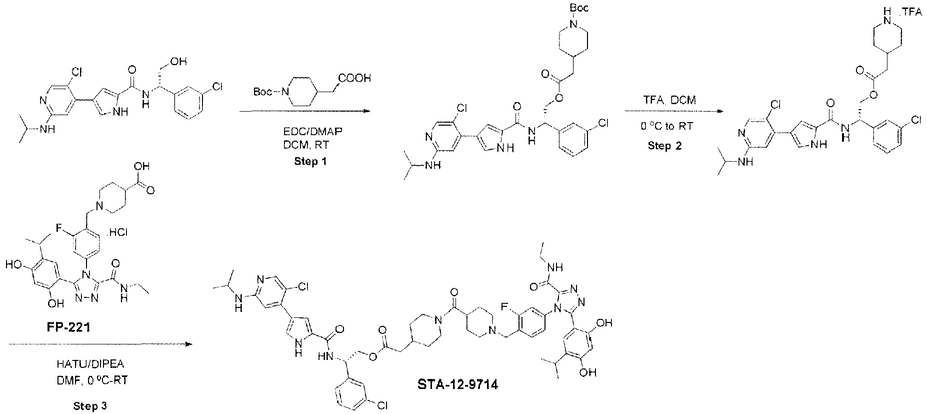

*Step 1:* To a mixture of (S)-4-(5-chloro-2-(isopropylamino)pyridin-4-yl)-N-(1-(3-chlorophenyl)-2-hydroxyethyl)-1H-pyrrole-2-carboxamide (65 mg, 0.15 mmol) and 2-(1-(tert-butoxycarbonyl)piperidin-4-yl)acetic acid (40 mg, 0.165 mmol) in dry CH₂Cl₂ (4 mL) was added EDC (32 mg, 0.165 mmol) and DMAP (20 mg, 0.165 mmol). The reaction mixture was stirred at room temperature overnight. The mixture was diluted with DCM and washed with aqueous NH₄Cl solution. The organic layer was dried over Na₂SO₄ and concentrated in vacuum. The crude residue was purified by ISCO using DCM/MeOH (0-10%) as eluent to afford 95 mg (96%) of product. ESMS calculated (C₃₃H₄)Cl₂N₅O₅): 658.62; found: 659.7 (M+H).

*Step 2:* To a solution of (S)-tert-butyl 4-(2-(2-(4-(5-chloro- 2-(isopropylamino)pyridin-4-yl)-1H-pyrrole-2-carboxamido)-2-(3-chlorophenyl)ethoxy)-2-oxoethyl)piperidine-1-carboxylate (47 mg, 0.071 mmol) in dry CH₂Cl₂ (1.6 mL) at 0 °C added TFA (0.4 mL). After 1.5 h, the ice bath removed and further stirred at room temperature for 30 min. The reaction mixture was concentrated, co-evaporated with hexanes several times and vacuum dried. The crude product was obtained as TFA salt and used in the next step without further purification.

*Step 3:* To a mixture of (S)-2-(4-(5-chloro-2-(isopropylamino)pyridin-4-yl)-1H-pyrrole-2-carboxamido)-2-(3-chlorophenyl)ethyl 2-(piperidin-4-yl)acetate (TFA salt) (47 mg, 0.071 mmol) and FP-221 (47 mg, 0.084 mmol) in DMF (3 mL) at 0 °C was added HATU (27 mg, 0.069 mmol) and DIPEA (0.1 mL, 0.57 mmol). After 1 h, the ice bath removed, further stirred at room temperature for 1 h. The crude mixture was treated with water, extracted with EtOAc. The organic layer was dried over Na₂SO₄ and concentrated in vacuum. The crude residue was purified by ISCO using DCM/MeOH (0-10%) as eluent to afford 64 mg (85%) of product. ¹H NMR (400 MHz, DMSO-d₆) δ 11.93 (s, 1H), 10.38 (s, 1H), 9.77(s, 1H), 9.01 (t, J = 6 Hz, 1H), 8.66 (t, J = 7.8 Hz, 1H), 7.99-7.98 (m, 1H), 7.53 (s, 1H), 7.41-7.33 (m, 7H), 7.15 (d, J = 8 Hz, 1H), 6.66 (s, 1H), 6.60 (s, 1H), 6.46 (t, J = 7.6 Hz, 1H), 6.33 (s, 1H), 5.46-5.35 (m, 1H), 4.40-4.20 (m, 3H), 4.0-3.90 (m, 1H), 3.80-3.54 (m, 1H), 3.48-3.39 (m, 2H), 3.21-3.14 (m, 2H), 2.97-2.90 (m, 1H), 2.85-2.70 (m, 2H), 2.67-2.55 (m, 1H), 2.39-2.08 (m, 4H), 2.05-1.70 (m, 3H), 1.68-1.35 (m, 6H), 1.13 (d, J = 6.4 Hz, 6H), 1.050 (t, J = 7.2 Hz, 3H), 0.96-0.90 (m, 2H), 0.86 (d, J = 6.8 Hz, 6H); ESMS calculated (C₅₅H₆₃Cl₂FN₁₀O₇): 1066.06; found: 1067 (M+H).

### SDC-TRAP-0513

### (8)-2-(4-(S-chloro-2-(isopropylamino)pyridin-4-yl)-1H-pyrrole-2-carboxamido)-2-(3-chlorophenyl)ethyl 2-(1-(1-(4-(3-(2,4-dihydroxy-5-isopropylphenyl)-5-hydroxy-4H-1,2,4-triazol-4-yl)-2-fluorohenzyl)piperidine-4-carbonyl)piperidin-4-yl)acetate

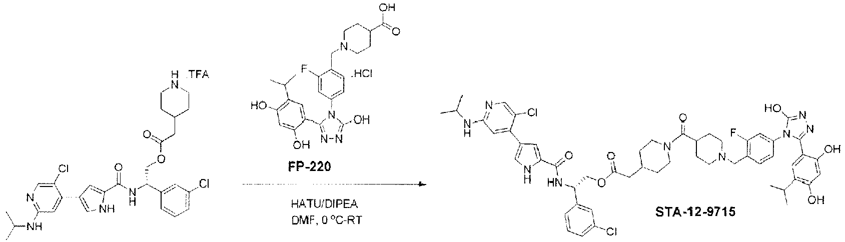

To a mixture of (S)-2-(4-(5-chloro-2-(isopropylamino)pyridin-4-yl)-1H-pyrrole-2-carboxamido)-2-(3-chlorophenyl)ethyl 2-(piperidin-4-yl)acetate (TFA salt) (47 mg, 0.071 mmol) and FP-220 (43 mg, 0.084 mmol) in DMF (3 mL) at 0 °C was added HATU (27 mg, 0.069 mmol) and DIPEA (0.1 mL, 0.57 mmol). After 1 h, the ice bath removed, further stirred at room temperature for 1 h. The crude mixture was treated with water, extracted with EtOAc. The organic layer was dried over Na₂SO₄ and concentrated in vacuum. The crude residue was purified by ISCO using DCM/MeOH (0-10%) as eluent to afford 52 mg (73%) of product. ¹H NMR (400 MHz, DMSO-d₆) δ 11.99 (s, 1H), 11.93 (s, 1H), 9.63 (s, 1H), 9.41 (s, 1H), 8.66 (broad s, 1H), 7.98 (s, 1H), 7.53 (s, 1H), 7.42-7.31 (m, 6H), 7.04 (d, J = 10.8 Hz, 1H), 6.96 (d, J = 8 Hz, 1H), 6.85 (s, 1H), 6.60 (s, 1H), 6.50-6.40 (m, 1H), 6.26 (s, 1H), 5.45-5.35 (m, 1H), 4.40-4.20 (m, 3H), 4.0-3.90 (m, 1H), 3.75-3.56 (m, 1H), 3.43 (broad s, 1H), 3.03-2.96 (m, 1H), 2.80-2.50 (m. 3H), 2.40-2.10 (m, 4H), 2.0-1.70 (m, 3H), 1.65-1.41 (m, 6H), 1.12 (d, J = 6 Hz, 6H), 0.96 (d, J = 6.8 Hz, 6H), 0.91-0.76 (m, 3H); ESMS calculated (C₅₅H₆₃Cl₂FN₁₀O₇): 1066.06; found: 1067 (M+H).

### SDC-TRAP-0514 (S)-2-(4-(5-chloro-2-(isopropylamino)pyridin-4-yl)-1H-pyrrole-2-carboxamido)-2-(3-chlorophenyl)ethyl 2-(1-(4-(3-(2,4-dihydroxy-5-isopropylphenyl)-5-(elhylcarbamoyl)-4H-1,2,4-triazol-4-yl)-2-fluorobenzyl)piperidine-4-carboxamido)-2-methylpropanoate

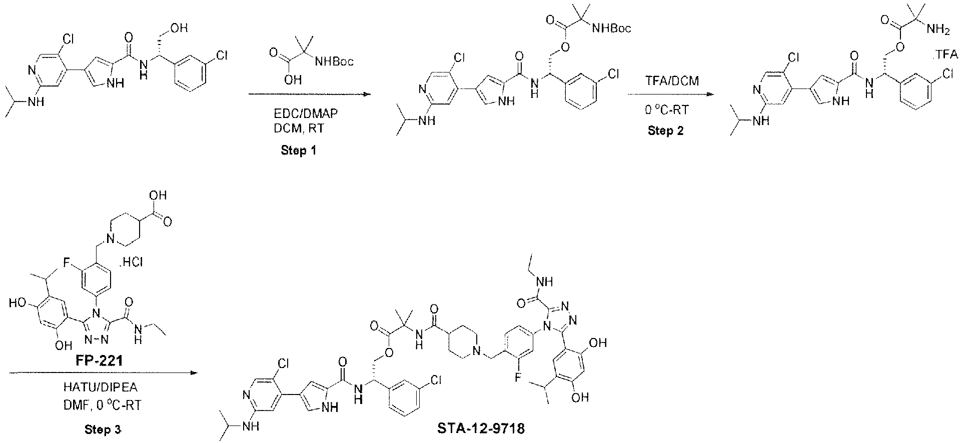

*Step 1:* To a mixture of (S)-4-(5-chloro-2-(isopropylamino)pyridin-4-yl)-N-(1-(3-chlorophenyl)-2-hydroxyethyl)-1H-pyrrole-2-carboxamide (44 mg, 0.1 mmol) and 2-((tert-butoxycarbonyl)amino)-2-methylpropanoic acid (23 mg, 0.11 mmol) in dry CH₂Cl₂ (4.5 mL) was added EDC (21 mg, 0.11 mmol) and DMAP (14 mg, 0.11 mmol). The reaction mixture was stirred at room temperature overnight. The mixture was diluted with DCM and washed with aqueous NH₄Cl solution. The organic layer was dried over Na₂SO₄ and concentrated in vacuum. The crude residue was purified by ISCO using DCM/MeOH (0-10%) as eluent to afford 59 mg (96%) of product. ESMS calculated (C₃₀H₃₇Cl₂N₅O₅): 618.55; found: 619 (M+H).

*Step 2:* To a solution of (S)-2-(4-(5-chloro-2-(isopropylamino)pyridin-4-yl)-1H-pyrrole-2-carboxamido)- 2-(3-chlorophenyl)ethyl 2-((tert-butoxycarbonyl)amino)-2-methylpropanoate (59 mg, 0.095 mmol) in dry CH₂Cl₂ (1.6 mL) at 0 °C added TFA (0.4 mL). After 45 min, the ice bath removed and further stirred at room temperature for 2 h. The reaction mixture was concentrated, co-evaporated with hexanes several times and vacuum dried. The crude product was obtained as TFA salt and used in the next step without further purification.

*Step 3:* To a mixture of (S)-2-(4-(5-chloro-2-(isopropylamino)pyridin-4-yl)-1H-pyrrole-2-carboxamido)-2-(3-chlorophenyl)ethyl 2-amino-2-methylpropanoate (TFA salt) (59 mg, 0.095 mmol) and FP-221 (64 mg, 0.114 mmol) in DMF (3 mL) at 0 °C was added HATU (36 mg, 0.095 mmol) and DIPEA (0.14 mL, 0.77 mmol). After 1 h, the ice bath removed, further stirred at room temperature for 1 h. The crude mixture was treated with water, extracted with EtOAc. The organic layer was dried over Na₂SO₄ and concentrated in vacuum. The crude residue was purified by ISCO using DCM/MeOH (0-10%) as eluent to afford 61 mg (63%) of product. ESMS calculated (C₅₂H₅₉Cl₂FN₁₀O₇): 1025.99; found: 1027 (M+H).

### SDC-TRAP-0515

### (S)-2-(4-(5-chloro-2-(isopropylamino)pyridin-4-yl)-1H-pyrrole-2-carboxamido)-2-(3-chlorophenyl)ethyl 1-(1-(4-(3-(2,4-dihydroxy-5-isopropylphenyl)-5-(ethylcarbamoyl)-4H-1,2,4-triazol-4-yl)-2-fluorobenzyl)piperidine-4-carbonyl)azetidine-3-carboxylale

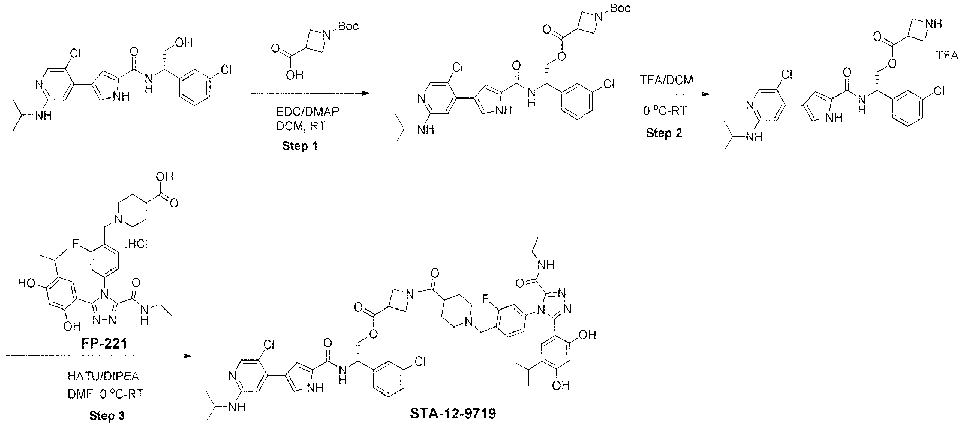

*Step 1:* To a mixture of (S)-4-(5-chloro-2-(isopropylamino)pyridin-4-yl)-N-(1-(3-chlorophenyl)-2-hydroxyethyl)-1H-pyrrole-2-carboxamide (44 mg, 0.1 mmol) and 1-(tert-butoxycarbonyl)azetidine-3-carboxylic acid (23 mg, 0.11 mmol) in dry CH₂Cl₂ (4.5 mL) was added EDC (21 mg, 0.11 mmol) and DMAP (14 mg, 0.11 mmol). The reaction mixture was stirred at room temperature overnight. The mixture was diluted with DCM and washed with aqueous NH₄Cl solution. The organic layer was dried over Na₂SO₄ and concentrated in vacuum. The crude residue was purified by ISCO using DCM/MeOH (0-10%) as eluent to afford 55 mg (90%) of product. ESMS calculated (C₃₀H₃₅Cl₂N₅O₅): 616.54; found: 617 (M+H).

*Step 2:* To a solution of (S)-1-tert-butyl 3-(2-(4-(5-chloro-2-(isopropylamino) pyridin-4-yl)-1H-pyrrole-2-carboxamido)-2-(3-chlorophenyl)ethyl)azetidine-1,3-dicarboxylate (55 mg, 0.089 mmol) in dry CH₂Cl₂ (1.6 mL) at 0 °C added TFA (0.4 mL). After 45 min, the ice bath removed and further stirred at room temperature for 2 h. The reaction mixture was concentrated, co-evaporated with hexanes several times and vacuum dried. The crude product was obtained as TFA salt and used in the next step without further purification.

*Step 3:* To a mixture of (S)-2-(4-(5-chloro-2-(isopropylamino)pyridin-4-yl)-1H-pyrrole-2-carboxamido)-2-(3-chlorophenyl)ethyl azetidine-3-carboxylate (TFA salt) (55 mg, 0.089 mmol) and FP-221 (60 mg, 0.107 mmol) in DMF (3 mL) at 0 °C was added HATU (34 mg, 0.089 mmol) and DIPEA (0.12 mL, 0.71 mmol). After 1 h, the ice bath removed, further stirred at room temperature for 1 h. The crude mixture was treated with water, extracted with EtOAc. The organic layer was dried over Na₂SO₄ and concentrated in vacuum. The crude residue was purified by ISCO using DCM/MeOH (0-10%) as eluent to afford 64 mg (70%) of product. ESMS calculated (C₅2H₅₇Cl₂FN₁₀O₇): 1023.98; found: 1025 (M+H).

### SDC-TRAP-0516

### (S)-(S)-2~(4-(5-chloro-2-(isopropylamino)pyridin-4-yl)-1H-pyrrole-2-carboxamido)-2-(3-chlorophenyl)ethyl 1-(1-(4-(3-(2,4-dihydroxy-5-isopropylphenyl)-5-(ethylcarbamoyl)-4H-1,2,4-triazol-4-yl)-2-fluorobenzyl)piperidine-4-carbonyl)pyrrolidine-3-carhoxylate

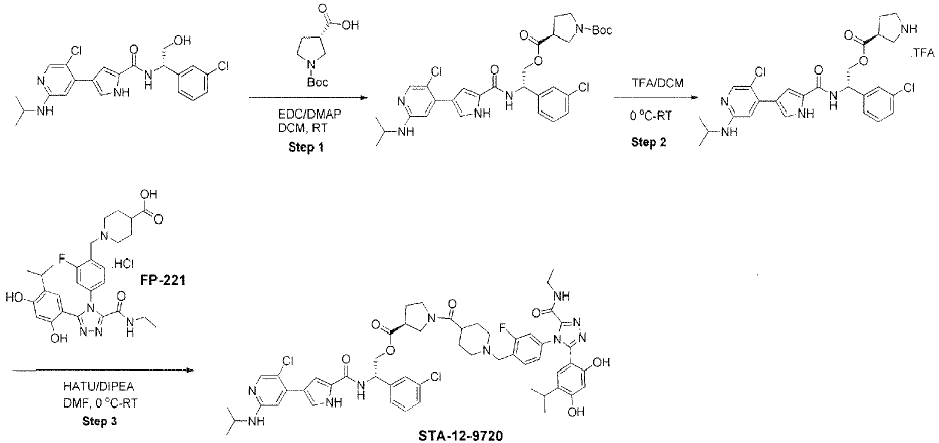

*Step 1:* To a mixture of (S)-4-(5-chloro-2-(isopropylamino)pyridin-4-yl)-N-(1-(3-chlorophenyl)-2-hydroxyethyl)-1H-pyrrole-2-carboxamide (44 mg, 0.1 mmol) and (S)-1-(tert-butoxycarbonyl)pyrrolidine-3-carboxylic acid (24 mg, 0.11 mmol) in dry CH₂Cl₂ (4.5 mL) was added EDC (21 mg, 0.11 mmol) and DMAP (14 mg, 0.11 mmol). The reaction mixture was stirred at room temperature overnight. The mixture was diluted with DCM and washed with aqueous NH₄Cl solution. The organic layer was dried over Na₂SO₄ and concentrated in vacuum. The crude residue was purified by ISCO using DCM/MeOH (0-10%) as eluent to afford 64 mg (99%) of product. ESMS calculated (C₃₁H₃₇Cl₂N₅O₅): 630.56; found: 631(M+H).

*Step 2:* To a solution of (S)-1-tert-butyl 3-((S)-2-(4-(5-chloro-2-(isopropylamino)pyridin-4-yl)-1H-pyrrole-2-carboxamido)-2-(3-chlorophenyl)ethyl) pyrrolidine-1,3-dicarboxylate (64 mg, 0.101 mmol) in dry CH₂Cl₂ (1.6 mL) at 0 °C added TFA (0.4 mL). After 45 min, the ice bath removed and further stirred at room temperature for 2 h. The reaction mixture was concentrated, co-evaporated with hexanes several times and vacuum dried. The crude product was obtained as TFA salt and used in the next step without further purification.

*Step 3:* To a mixture of (S)-(S)-2-(4-(5-chloro-2-(isopropylamino)pyridin-4-yl)-1H-pyrrole-2-carboxamido)-2-(3-chlorophenyl)ethyl pyrrolidine-3-carboxylate (TFA salt) (64 mg, 0.101 mmol) and FP-221 (68 mg, 0.121 mmol) in DMF (3 mL) at 0 °C was added HATU (38 mg, 0.101 mmol) and DIPEA (0.14 mL, 0.8 mmol). After 1 h, the ice bath removed, further stirred at room temperature for 1 h. The crude mixture was treated with water, extracted with EtOAc. The organic layer was dried over Na₂SO₄ and concentrated in vacuum. The crude residue was purified by ISCO using DCM/MeOH (0-10%) as eluent to afford 70 mg (67%) of product. ESMS calculated (C₅₃H₅₉Cl₂FN₁₀O₇): 1038; found: 1039 (M+H).

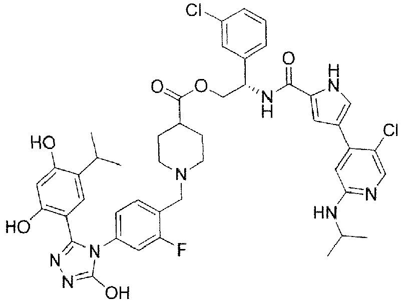

### SDC-TRAP-0517

### (8)-2-(4-(5-chloro-2-(isopropylamino)pyridin-4-yl)-1H-pyrrole-2-carboxamido)-2-(3-chlorophenyl)ethyl 1-(4-(3-(2,4-dihydroxy-5-isopropylphenyf)-5-hydroxy-4H-1,2,4-triazol-4-yl)-2-fluoro benzyl)piperidine-4-carboxylate.

Following the general procedure to SDC-TRAP-0507, SDC-TRAP-0517 was prepared; ESMS calculated (C₄₅H₄₇Cl₂FN₈O₆): 884.3; found: 885.2 (M+H).

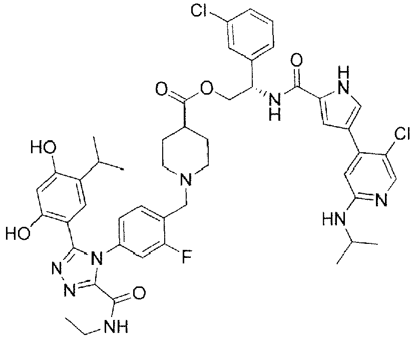

### SDC-TRAP-0518

### (S)-2-(4-(5-chloro-2-(isopropylamino)pyridin-4-yl)-1H-pyrrole-2-carboxamido)-2-(3-chlorophenyl)ethyl 1-(4-(3-(2,4-dihydroxy-5-isopropylphenyl)-5-(ethylcarbamoyl)-4H-1,2,4-triazol-4-yl)-2-fluorobenzyl)piperidine-4-carboxylate.

Following the general procedure to SDC-TRAP-0507, SDC-TRAP-0518 was obtained; ¹H NMR (400 MHz, DMSO) δ 11.92 (s, 1H), 10.39 (s, 1H), 9.77 (s, 1H), 9.00 (t, *J* = 5.6 Hz, 1H), 8.66 (d, *J* = 8.8 Hz, 1H), 7.96 (s, 1H), 7.95 (s, 2H) 7.52 (s, 1H), 7.41-7.31 (m, 5H), 7.13 (dd, *J* = 8.0, 1.6 Hz, 1H), 6.64 (s, 1H), 6.59 (s, 1H), 6.43 (d, *J* = 8.0 Hz, 1H), 6.34 (s, 1H), 5.36 (dd, *J* = 14.8, 8.0 Hz, 1H), 4.34-4.28 (m, 2H), 3.99-3.90 (m, 1H), 3.21-3.14 (m, 1H), 2.98-2.92 (m, 2H), 2.69-2.66 (m, 2H), 2.33-2.27 (m, 1H), 2.05-2.00 (m, 2H), 1.72-1.50 (m, 4H), 1.23-1.12 (m, 8H), 1.04 (t, *J* = 7.2 Hz, 3H), 0.84 (d, *J* = 6.8 Hz, 6H); ESMS calculated (C₄₈H₅₂Cl₂FN₉O₆): 939.3; found: 940.3 (M+H).

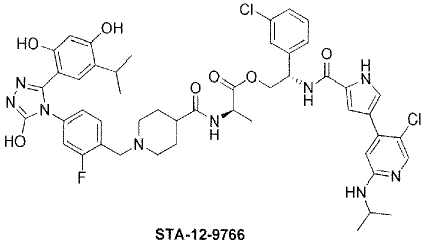

### SDC-TRAP-0519 (R)-(S)-2-(4-(5-chloro-2-(isopropylamino)pyridin-4-yl)-1H-pyrrole-2-carboxamido)-2-(3-chlorophenyl)ethyl 2-(1-(4-(3-(2,4-dihydroxy-5-isopropylphenyl)-5-hydroxy-4H-1,2,4-triazol-4-yl)-2-fluorobenzyl)piperidine-4-carboxamido)propanoate.

Following the general procedure to SDC-TRAP-0507, SDC-TRAP-0519 was prepared; ESMS calculated (C₄₈H₅₂Cl₂FN₉O₇): 955.3; found: 956.3 (M+H).

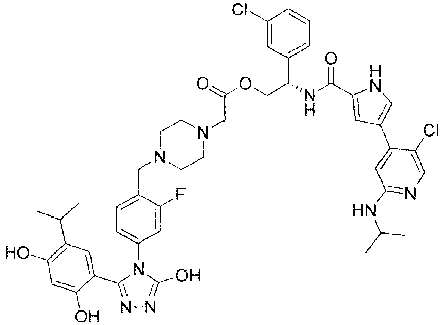

### SDC-TRAP-0520

### (S)-2-(4-(5-chloro-2-(isopropylamino)pyridin-4-yl)-1H-pyrrole-2-carboxamido)-2-(3-chlorophenyl)ethyl 2-(4-(4-(3-(2,4-dihydroxy-5-isopropylphenyl)-5-hydroxy-4H-1,2,4-tritizol-4-yl)-2-fluorobenzyl)piperazin-1-yl)acelate.

Following the general procedure to SDC-TRAP-0507, SDC-TRAP-0520 was prepared; ¹H NMR (400 MHz, DMSO) δ 11.99 (s, 1H), 11.90 (s, 1H), 9.63 (s, 1H), 9.40 (s, 1H), 8.66 (d, *J* = 8.8 Hz, 1H), 7.97 (s, 1H), 7.52 (s, 1 H), 7.41-7.27 (m, 6H), 7.04 (dd, *J* = 10.8, 2.0 Hz, 1H), 6.94 (dd, *J* = 8.4, 2.0 Hz, 1H), 6.87 (s, 1H), 6.60 (s, 1H), 6.43 (d, *J* = 7.6 Hz, 1H), 6.27 (s, 1H), 5.39-5.33 (m, 1H), 4.38-4.28 (m, 2H), 3.99-3.90 (m, 1H), 3.40 (s, 2H), 3.17 (s, 2H), 3.04-2.97 (m, 1H), 2.45-2.20 (m, 8H), 1.13 (d, *J* = 6.4 Hz, 6H), 1.00 (d, *J =* 6.4 Hz, 6H); ESMS calculated (C₄₅H₄₈Cl₂FN₉O₆): 899.3; found: 900.2 (M+H).

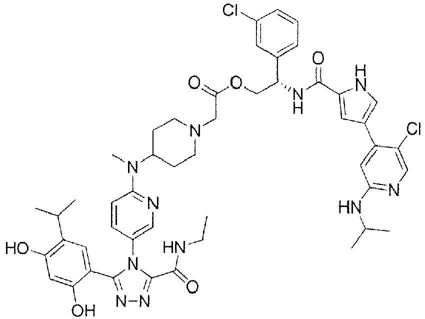

### SDC-TRAP-0521

### (S)-2-(4-(5-chloro-2-(isopropylamino-1H-pyrrole-2-carboxamido)-2-(3-chlorophenyl)ethyl 2-(4-((5-(3-(2,4-dihydroxy-5-isopropylphenyl)-5-(ethylcarbamoyl)-4H-1,2,4-triazol-4-yl)pyridin-2-yl)(methyl)amino)piperidin-1-yl)acetate.

Following the general procedure to SDC-TRAP-0507, SDC-TRAP-0521 was prepared; ESMS calculated (C₄₈H₅₅Cl₂N₁₁O₆): 951.4; found: 952.3 (M+H).

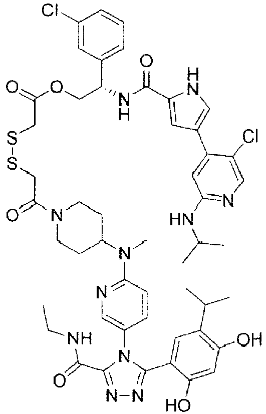

### SDC-TRAP-0522 (S)-2-(4-(5-chloro-2-(isopropylamino)pyridin-4-yl)-1H-pyrrole-2-carboxamido)-2-(3-chlorophenyl)ethyl 2-((2-(4-((5-(3-(2,4-dihydroxy-5-isopropylphenyl)-5-(ethylcarbamoyl)-4H-1,2,4-triazol-4-yl)pyridin-2-yl)(melhyl)amino)piperidin-1-yl)-2-oxoethyl)disulfanyl)acetate.

Following the general procedure to SDC-TRAP-0517, SDC-TRAP-0522 was prepared; ESMS calculated (C₅₀H₅₇Cl₂N₁₁O₇S₂): 1057.3; found: 1058.1 (M+H).

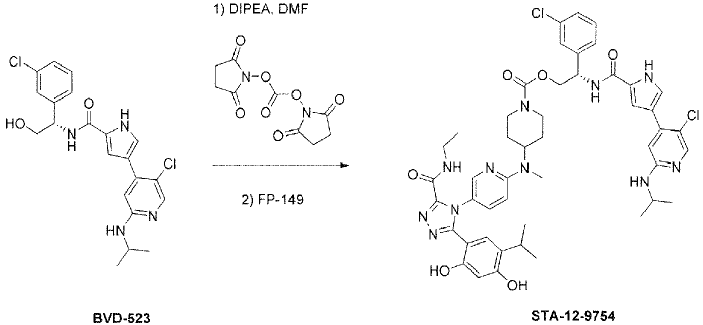

### SDC-TRAP-0523

### (S)-2-(4-(5-chloro-2-(isopropylamino)pyridin-4-yl)-1H-pyrrole-2-carboxamido)-2-(3-chlorophenyl)ethyl 4-((5-(3-(2,4-dihydroxy-5-isopropylphenyl)-5-(ethylcarbamoyl)-4H-1,2,4-triazol-4-yl)pyridin-2-yl)(methyl)amino)piperidine-1-carboxylate.

To the solution of BVD-523 (0.05 g, 0.12 mmol) in DMF (2 mL) was added disuccinimidyl carbonate (0.033 g, 0.13 mmol) and DIPEA (0.06 mL, 0.36 mmol). The reaction was stirred at room temperature for 3 hr before FP-149 (0.06 g, 0.13 mmol) was added. The resulting solution was stirred at room temperature for another 1 hr. The reaction solution was diluted with H₂O (2 mL) and extracted with EtOAc (5 mLX3). The organic phase was separated, dried over Na₂SO₄ and concentrated. The column chromatography (DCM/MeOH) gave SDC-TRAP-0523 (0.057 g, 51%). ESMS calculated (C₄₇H₅₃Cl₂N₁₁O₆): 937.4; found: 938.2 (M+H).

### Example 2- Hsp90 inhibitor MEK kinase inhibitor (TAK-733) conjugates

MEK inhibitor TAK-733 selectively bids to and inhibits the activity of MEK1/2, preventing the activation of MEK1/2-dependent effector proteins and transcription factors.

General procedure for MEKi ester conjugation:

### SDC-TRAP-0524

### (R)-3-(6-fluoro-5-((2-fluoro-4-iodophenyl)amino)-8-methyl-4,7-dioxo-7,8-dihydropyrido[2,3-d]pyrimidin-3(4H)-yl)-2-hydroxypropyl 4-(4-(4-(3-(2,4-dihydroxy-5-isopropylphenyl)-5-hydroxy-4H-1,2,4-triazol-4-yl)-2-fluorobenzyl)piperazin-1-yl)-4-oxobutanoate

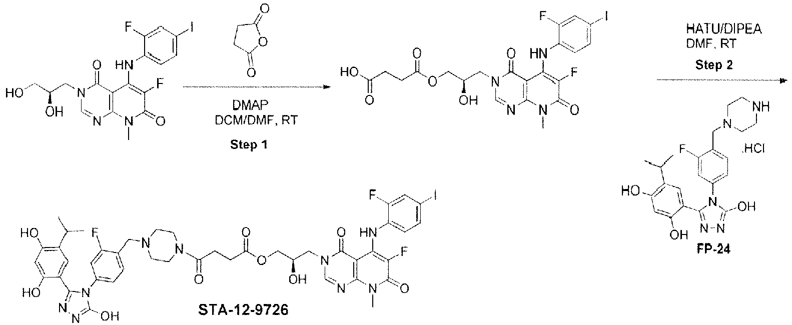

*Step 1:* To a mixture of (R)-3-(2,3-dihydroxypropyl)-6-fluoro-5-((2-fluoro-4-iodophenyl)amino)-8-methylpyrido[2,3-d]pyrimidine-4,7(3H,8H)-dione (25 mg, 0.05 mmol) and dihydrofuran-2,5-dione (6 mg, 0.06 mmol) in dry DCM/DMF (2 mL/0.5 mL) was added DMAP (7.4 mg, 0.06 mmol). The mixture was stirred at room temperature overnight, diluted with EtOAc and washed with 0.1 N aq. HCl. The organic layer was dried over Na₂SO₄ and concentrated in vacuum. The crude product was used in the next step without further purification. ESMS calculated (C₂₁H₁₉F₂IN₄O₇): 604.30; found: 605 (M+H).

*Step 2:* To a mixture of (R)-4-(3-(6-fluoro-5-((2-fluoro-4-iodophenyl)amino)-8-methyl-4,7-dioxo-7,8-dihydropyrido[2,3-d]pyrimidin-3(4H)-yl)-2-hydroxypropoxy)-4-oxobutanoic acid (30 mg, 0.05 mmol) and FP-24 (28 mg, 0.06 mmol) in DMF (1 mL) was added HATU (19 mg, 0.05 mmol) and DIPEA (0.035 mL, 0.2 mmol). The reaction mixture was stirred at room temperature for 3 h and treated with water. The resulting solid was filtered, dried, purified by ISCO using DCM/MeOH (0-10%) as eluent to afford 8.7 mg (17%) of SDC-TRAP-0524. ¹H NMR (400 MHz, DMSO-d6) δ 11.99 (s, 1H), 10.19 (s, 1H), 9.63 (s, 1H), 9.40 (s, 1H), 8.54 (s, 1H), 7.70-7.67 (m, 1H), 7.50 (d, J = 8 Hz, 1H), 7.37 (t, J = 8 Hz, 1H), 7.07-6.95 (m, 3H), 6.87 (s, 1H), 6.26 (s, 1H), 5.50 (s, 1H), 4.23-4.20 (m, 1H), 4.01 (s, 3H), 3.83-3.77 (m, 1H), 3.58 (s, 3H), 3.49 (s, 2H), 3.40 (broad s, 4H), 3.033-2.96 (m, 1H), 2.56-2.55 (m, 2H), 2.39-2.34 (m, 2H), 2.31-2.25 (m, 2H), 1.23 (s, 2H), 1.00 (s, J = 6.8 Hz, 6H); ESMS calculated (C₄₃H₄₃F₃IN₉O₉): 1013.76; found: 1014 (M+H).

### SDC-TRAP-0525

### (R)-3-(6-fluoro-5-((2-fluoro-4-iodophenyl)amino)-8-methyl-4,7-dioxo-7,8-dihydropyrido[2,3-d]pyrimidin-3(4H)-yl)-2-hydroxypropyl 4-(4-((5-(3-(2,4-dihydroxy-5-isopropylphenyl)-5-(ethylcarbamoyl)-4H-1,2,4-triazol-4-yl)pyridin-2-yl)(methyl)amino)piperidin-1-yl)-4-oxobutanoate

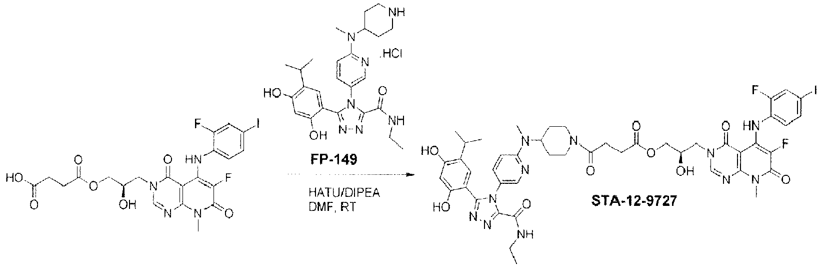

To a mixture of (R)-4-(3-(6-fluoro-5-((2-fluoro-4-iodophenyl)amino)-8-methyl-4,7-dioxo-7,8-dihydropyrido[2,3-d]pyrimidin-3(4H)-yl)-2-hydroxypropoxy)-4-oxobutanoic acid (78 mg, 0.129 mmol) and FP-149 (66 mg, 0.129 mmol) in DMF (2 mL) was added HATU (49 mg, 0.129 mmol) and DIPEA (0.090 mL, 0.52 mmol). The reaction mixture was stirred at room temperature for 1.5 h and treated with water. The resulting solid was filtered, dried, purified by ISCO using DCM/MeOH (0-10%) as eluent to afford 69 mg (50%) of title compound. ¹H NMR (400 MHz, DMSO-d6) δ 10.29 (s, 1H), 10.20 (s, 1H), 9.75 (s, 1H), 8.98 (t, J = 6 Hz, 1H), 8.55 (s, 1H), 7.96-7.95 (m, 1H), 7.70-7.67 (m, 1H), 7.53-7.46 (m, 2H), 7.02-6.96 (m, 1H), 6.7 (s, 1H), 6.67 (d, J = 8 Hz, 1H), 6.33 (s, 1H), 5.51 (s, 1H), 4.71 (broad s, 1H), 4.49-4.46 (m, 1H), 4.25-4.22 (m, 1H), 4.04 (s, 3H), 3.98-3.95 (m, 1H), 3.85-3.80 (m, 1H), 3.59 (s, 3H), 3.20-3.09 (m, 3H), 3.0-2.93 (m, 1H), 2.81 (s, 3H), 2.70-2.54 (m, 5H), 1.71-1.50 (m, 4H), 1.06 (t, J = 8 Hz, 3H), 0.92 (d, J = 8 Hz, 6H); ESMS calculated for C₄₆H₅₀F₂IN₁₁O₉: 1065.86; found: 1066.4 (M+H).

### SDC-TRAP-0526

### (S)-(R)-3-(6-fluoro-5-((2-fluoro-4-iodophenyl)amino)-8-methyl-4,7-dioxo-7,8-dihydropyrido[2,3-d]pyrimidin-3(4H)-yl)-2-hydroxypropyl 2-(4-(3-(2,4-dihydroxy-5-isopropylphenyl)-5-(ethylcarbamoyl)-4H-1,2,4-triazol-4-yl)benzamido)-3-methylbutanoate

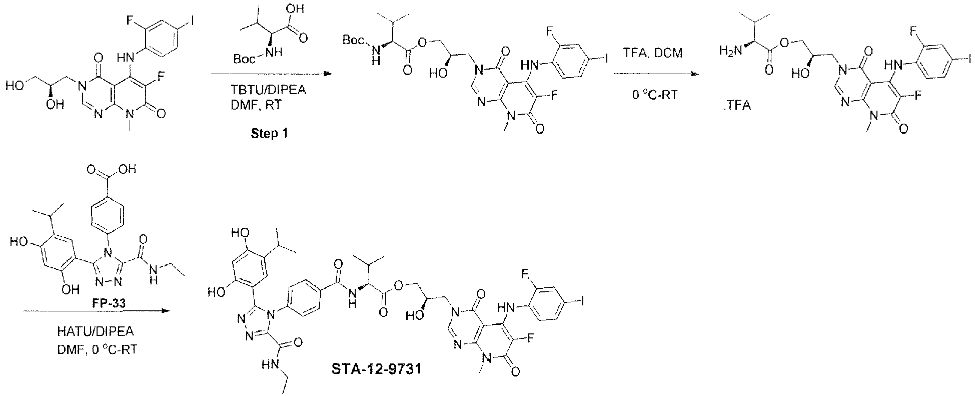

*Step 1:* (S)-2-((tert-butoxycarbonyl)amino)-3-methylbutanoic acid (52 mg, 0.24 mmol), TBTU (77 mg, 0.24 mmol) and DIPEA (0.087 mL, 0.495 mmol) were dissolved in dry DMF (1 mL) and the resulting mixture stirred at room temperature for 30 min. To the above mixture (R)-3-(2,3-dihydroxypropyl)-6-fluoro-5-((2-fluoro-4-iodophenyl)amino)-8-methylpyrido[2,3-d]pyrimidine-4,7(3H,8H)-dione (100 mg, 0.198 mmol) was added and stirred at room temperature for 20 h. The reaction mixture was diluted with the EtOAC and washed with brine. The organic layer was dried over Na₂SO₄ and concentrated in vacuum. The crude residue was purified by ISCO using DCM/MeOH (0-10%) as eluent to afford 110 mg (63%) of product. ESMS calculated (C₂₇H₃₂F₂IN₅O₇): 703.47; found: 704 (M+H).

*Step 2:* To a solution (S)-(R)-3-(6-fluoro-5-((2-fluoro-4-iodophenyl)amino)- 8-methyl-4,7-dioxo-7,8-dihydropyrido[2,3-d]pyrimidin-3(4H)-yl)-2-hydroxypropyl 2-((tert-butoxycarbonyl)amino)-3-methylbutanoate (100 mg, 0.142 mmol) in dry CH₂Cl₂ (2.4 mL) at 0 °C added TFA (0.6 mL). After 15 min, the ice bath removed and further stirred at room temperature for 2 h. The reaction mixture was concentrated, co-evaporated with hexanes several times and vacuum dried. The crude product was obtained as TFA salt and used in the next step without further purification.

*Step 3:* To a mixture of (S)-(R)-3-(6-fluoro-5-((2-fluoro-4-iodophenyl)amino)-8-methyl-4,7-dioxo-7,8-dihydropyrido[2,3-d]pyrimidin-3(4H)-yl)-2-hydroxypropyl 2-amino-3-methylbutanoate (TFA salt) (100 mg, 0.142 mmol) and FP-33 (73 mg, 0.17 mmol) in DMF (5 mL) at 0 °C was added HATU (54 mg, 0.142 mmol) and DIPEA (0.2 mL, 1.136 mmol). After 30 min, the ice bath removed, further stirred at room temperature for 1.5 h. The crude mixture was treated with water, extracted with EtOAc. The organic layer was dried over Na₂SO₄ and concentrated in vacuum. The crude residue was purified by ISCO using DCM/MeOH (0-10%) as eluent to afford 103 mg (73%) of title compound. ESMS calculated (C₄₃H₄₄F₂IN₉O₉): 995.77; found: 996 (M+H).

### SDC-TRAP-0527

### (R)-3-(6-fluoro-5-((2-fluoro-4-iodophenyl)amino)-8-methyl-4,7-dioxo-7,8-dihydropyrido[2,3-d]pyrimidin-3(4H)-yl)-2-hydroxypropyl 2-(4-(3-(2,4-dihydroxy-5-isopropylphenyl)-5-(ethylcarbamoyl)-4H-1,2,4-triazol-4-y)-N-methylbenzamido)acetate

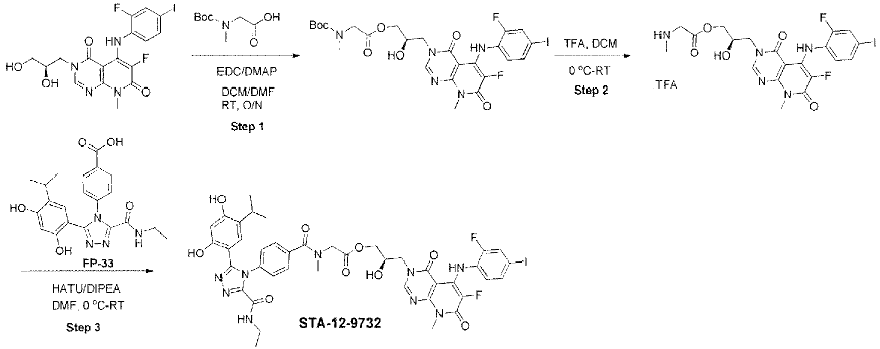

*Step 1:* To a mixture of (R)-3-(2,3-dihydroxypropyl)-6-fluoro-5-((2-fluoro-4-iodophenyl)amino)-8-methylpyrido[2,3-d]pyrimidine-4,7(3H,8H)-dione (100 mg, 0.198 mmol) and 2-((tert-butoxycarbonyl)(methyl)amino)acetic acid (42 mg, 0.22 mmol) in dry CH₂Cl₂ (2 mL) was added EDC (42 mg, 0.22 mmol) and DMAP (27 mg, 0.22 mmol). The reaction mixture was stirred at room temperature overnight. The mixture was diluted with EtOAc and washed with aqueous NH₄Cl solution. The organic layer was dried over Na₂SO₄ and concentrated in vacuum. The crude residue was purified by ISCO using DCM/MeOH (0-10%) as eluent to afford 40 mg (30%) of product. ESMS calculated (C₂₅H₂₈F₂IN₅O₇): 675.42; found: 676 (M+H).

*Step 2:* To a solution (R)-3-(6-fluoro-5-((2-fluoro-4-iodophenyl)amino)-8-methyl-4,7-dioxo-7,8-dihydropyrido[2,3-d]pyrimidin-3(4H)-yl)-2-hydroxypropyl 2-((tert-butoxycarbonyl)(methyl)amino)acetate (40 mg, 0.059 mmol) in dry CH₂Cl₂ (0.96 mL) at 0 °C added TFA (0.24 mL). After 15 min, the ice bath removed and further stirred at room temperature for 2 h. The reaction mixture was concentrated, co-evaporated with hexanes several times and vacuum dried. The crude product was obtained as TFA salt and used in the next step without further purification.

*Step 3:* To a mixture of (R)-3-(6-fluoro-5-((2-fluoro-4-iodophenyl)amino)-8-methyl-4,7-dioxo-7,8-dihydropyrido[2,3-d]pyrimidin-3(4H)-yl)-2-hydroxypropyl 2-(methylamino)acetate (TFA salt) (40 mg, 0.059 mmol) and FP-33 (31 mg, 0.07 mmol) in DMF (3 mL) at 0 °C was added HATU (23 mg, 0.059 mmol) and DIPEA (0.083 mL, 0.48 mmol). After 30 min, the ice bath removed, further stirred at room temperature for 1.5 h. The crude mixture was treated with water, extracted with EtOAc. The organic layer was dried over Na₂SO₄ and concentrated in vacuum. The crude residue was purified by ISCO using DCM/MeOH (0-10%) as eluent to afford 29 mg (50%) of title compound. ESMS calculated (C₄₁H₄₀F₂IN₉O₉): 967.71; found: 968 (M+H).

### SDC-TRAP-0528

### (S)-(R)-3-(6-fluoro-5-((2-fluoro-4-iodophenyl)amino)-8-methyl-4,7-dioxo-7,8-dihydropyrido[2,3-d]pyrimidin-3(4H)-yl)-2-hydroxypropyl 1-(1-(4-(3-(2,4-dihydroxy-5-isopropylphenyl)-5-hydroxy-4H-1,2,4-triazol-4-yl)-2-fluorobenzyl)piperidine-4-carbonyl)pyrrolidine-3-carboxylate

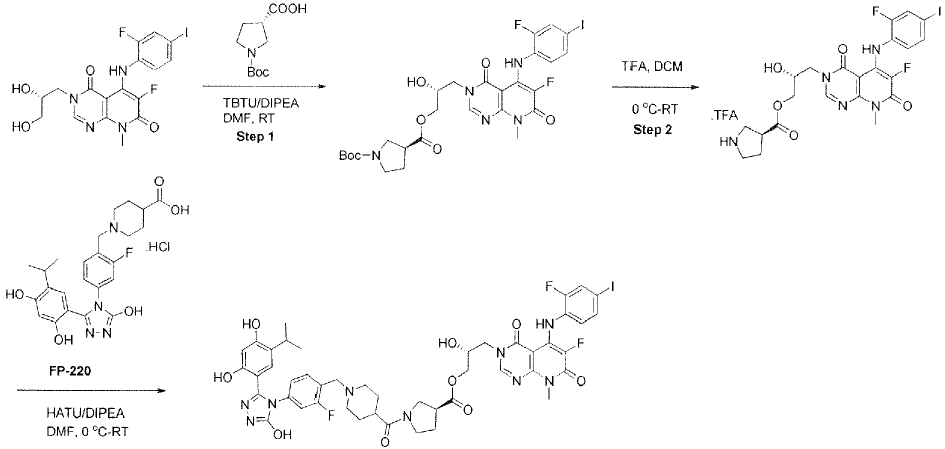

*Step 1:* (S)-1-(tert-butoxycarbonyl)pyrrolidine-3-carboxylic acid (52 mg, 0.24 mmol), TBTU (77 mg, 0.24 mmol) and DIPEA (0.14 mL, 0.8 mmol) were dissolved in dry DMF (1.5 mL) and the resulting mixture stirred at room temperature for 30 min. To the above mixture (R)-3-(2,3-dihydroxypropyl)-6-fluoro-5-((2-fluoro-4-iodophenyl)amino)-8-methylpyrido[2,3-d]pyrimidine-4,7(3H,8H)-dione (100 mg, 0.198 mmol) was added and stirred at room temperature for 20 h. The reaction mixture was diluted with the EtOAC and washed with brine. The organic layer was dried over Na₂SO₄ and concentrated in vacuum. The crude residue was purified by ISCO using DCM/MeOH (0-10%) as eluent to afford 50 mg (36.2%) of product. ESMS calculated (C₂₇H₃₀F₂IN₅O₇): 701.46; found: 702 (M+H).

*Step 2:* To a (S)-1-tert-butyl 3-((R)-3-(6-fluoro-5-((2-fluoro-4-iodophenyl)amino)-8-methyl-4,7-dioxo-7,8-dihydropyrido[2,3-d]pyrimidin-3(4H)-yl)-2-hydroxypropyl)pyrrolidine-1,3-dicarboxylate (50 mg, 0.071 mmol) in dry CH₂Cl₂ (1.2 mL) at 0 °C added TFA (0.3 mL). After 15 min, the ice bath removed and further stirred at room temperature for 2 h. The reaction mixture was concentrated, co-evaporated with hexanes several times and vacuum dried. The crude product was obtained as TFA salt and used in the next step without further purification.

*Step 3:* To a mixture of (S)-(R)-3-(6-fluoro-5- ((2-fluoro-4-iodophenyl)amino)-8-methyl-4,7-dioxo-7,8-dihydropyrido[2,3-d]pyrimidin-3(4H)-yl)-2-hydroxypropyl pyrrolidine-3-carboxylate (TFA salt) (50 mg, 0.071 mmol) and FP-220 (44 mg, 0.086 mmol) in DMF (3 mL) at 0 °C was added HATU (27 mg, 0.071 mmol) and DIPEA (0.10 mL, 0.57 mmol). After 30 min, the ice bath removed, further stirred at room temperature for 1.5 h. The crude mixture was treated with water, extracted with EtOAc. The organic layer was dried over Na₂SO₄ and concentrated in vacuum. The crude residue was purified by ISCO using DCM/MeOH (0-10%) as eluent to afford 55 mg (61.7%) of title compound. ESMS calculated (C₄₆H₄₇F₃IN₉O₉): 1053.82; found: 1054 (M+H).

### SDC-TRAP-0529

### (S)-(R)-3-(6-fluoro-5-((2-fluoro-4-iodophenyl)amino)-8-methyl-4,7-dioxo-7,8-dihydropyrido[2,3-d]pyrimidin-3(4H)-yl)-2-hydroxypropyl 2-(4-(3-(2,4-dihydroxy-5-isopropylphenyl)-5-(ethylcarbamoyl)-4H-1,2,4-triazol-4-yl)benzamido)propanoate

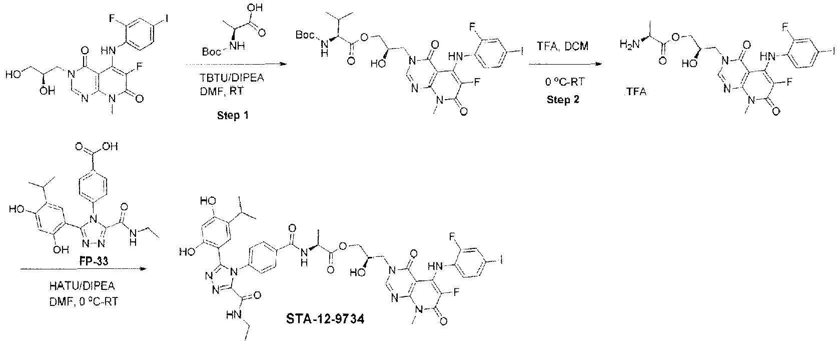

*Step 1:* (S)-2-((tert-butoxycarbonyl)amino)propanoic acid (38 mg, 0.198 mmol), TBTU (64 mg, 0.198 mmol) and DIPEA (0.069 mL, 0.4 mmol) were dissolved in dry DMF (1 mL) and the resulting mixture stirred at room temperature for 30 min. To the above mixture (R)-3-(2,3-dihydroxypropyl)-6-fluoro-5-((2-fluoro-4-iodophenyl)amino)-8-methylpyrido[2,3-d]pyrimidine-4,7(3H,8H)-dione (100 mg, 0.198 mmol) was added and stirred at room temperature for 20 h. The reaction mixture was diluted with the EtOAC and washed with brine. The organic layer was dried over Na₂SO₄ and concentrated in vacuum. The crude residue was purified by ISCO using DCM/MeOH (0-10%) as eluent to afford 63 mg (47.3%) of product. ESMS calculated (C₂₅H₂₈F₂IN₅O₇): 675.42: found: 676 (M+H).

*Step 2:* To a (S)-(R)-3-(6-fluoro-5-((2-fluoro-4-iodophenyl)amino)- 8-methyl-4,7-dioxo-7,8-dihydropyrido[2,3-d]pyrimidin-3(4H)-yl)-2-hydroxypropyl 2-((tert-butoxycarbonyl)amino)-3-methylbutanoate (63 mg, 0.093 mmol) in dry CH₂Cl₂ (1.6 mL) at 0 °C added TFA (0.4 mL). After 15 min, the ice bath removed and further stirred at room temperature for 2 h. The reaction mixture was concentrated, co-evaporated with hexanes several times and vacuum dried. The crude product was obtained as TFA salt and used in the next step without further purification.

*Step 3:* To a mixture of (S)-(R)-3-(6-fluoro-5-((2-fluoro-4-iodophenyl)amino)- 8-methyl-4,7-dioxo-7,8-dihydropyrido[2,3-d]pyrimidin-3(4H)-yl)-2-hydroxypropyl 2-aminopropanoate (TFA salt) (63 mg, 0.093 mmol) and FP-33 (48 mg, 0.116 mmol) in DMF (5 mL) at 0 °C was added HATU (36 mg, 0.093 mmol) and DIPEA (0.13 mL, 0.75 mmol). After 30 min, the ice bath removed, further stirred at room temperature for 1.5 h. The crude mixture was treated with water, extracted with EtOAc. The organic layer was dried over Na₂SO₄ and concentrated in vacuum. The crude residue was purified by ISCO using DCM/MeOH (0-10%) as eluent to afford 55 mg (61.7%) of title compound. ESMS calculated (C₄₁H₄₀F₂IN₉O₉): 967.71; found: 968 (M+H).

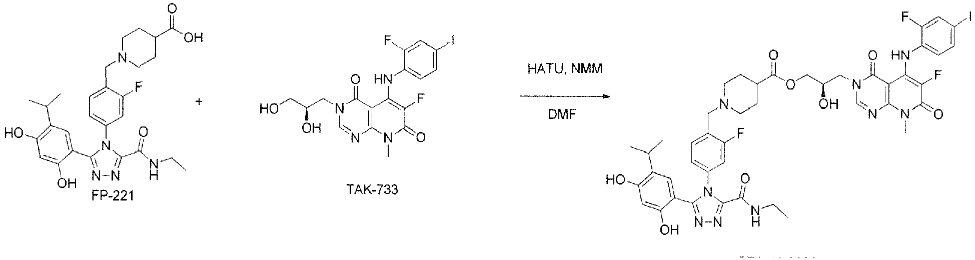

### SDC-TRAP-0530

### (R)-3-(6-fluoro-5-((2-fluoro-4-iodophenyl)amino)-8-methyl-4,7-dioxo-7,8-dihydropyrido[2,3-d]pyrimidin-3(4H)-yl)-2-hydroxypropyl 1-(4-(3-(2,4-dihydroxy-5-isopropylphenyl)-5-(ethylcarbamoyl)-4H-1,2,4-triazol-4-yl)-2-fluorobenzyl)piperidine-4-carboxylate.

To a solution of FP-221 (0.056 g, 0.10 mmol) in DMF (3 mL) were added TAK-733 (0.05 g, 0.10 mmol) and HATU (0.054 g, 0.14 mmol). The mixture was cooled to 0°C and NMM (0.06 mL, 0.5 mmol) was added. The mixture was stirred overnight at RT. The reaction mixture was diluted with EtOAc, washed with water and saturated aq. NaCl. The organic layer was dried over Na₂SO₄ and concentrated under reduced pressure. The residue was purified by flash column chromatography on silica gel (DMC/MeOH 98:2 to 9:1) to give compound SDC-TRAP-0530 as white solid (21mg, 21% yield). ¹H NMR (400 MHz, DMSO) δ 10.37 (s, 1H), 10.19 (s, 1H), 9.77 (s, 1H), 9.01 (s, 1H), 8.54 (s, 1H), 7.66 (s, 1H), 7.53 (d, *J* = 1.6 Hz, 1H), 7.44 (d, *J* = 8.8 Hz, 1H), 7.32 (d, *J* = 1.6 Hz, 1H), 7.22 (d, *J* = 1.6 Hz, 1H), 6.98 (dd, *J* = 8.4, 2.0 Hz, 1H), 6.66 (s, 1H), 6.33 (s, 1H), 5.52 (s, 1H), 4.24-3.85(m, 4H), 3.59 (s, 3H),3.19-2.76(m, 6H), 2.32-1.60 (m, 7H), 1.03 (t, *J* = 4.8 Hz, 3H), 0.86 (d, *J* = 8.8 Hz, 6H); ESMS calculated for C₄₄H₄₅F₃IN₉O₈: 1011.24; Found 1012.0 (M+H).

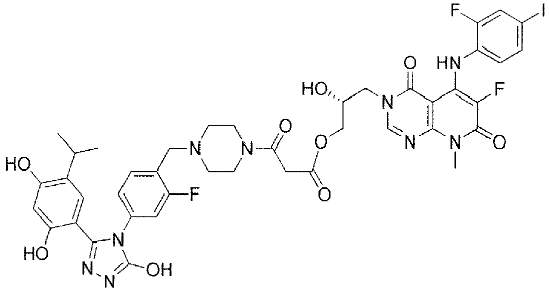

### SDC-TRAP-0531

### (R)-3-(6-fluoro-5-((2-fluoro-4-iodophenyl)amino)-8-methyl-4,7-dioxo-7,8-dihydropyrido[2,3-d]pyrimidin-3(4H)-yl)-2-hydroxypropyl 3-(4-(4-(3-(2,4-dihydroxy-5-isopropylphenyl)-5-hydroxy-4H-1,2,4-triazol-4-yl)-2-fluorobenzyl)piperazin-1-yl)-3-oxopropanoate.

Following the general procedure to SDC-TRAP-0524, SDC-TRAP-0531 was prepared; ESMS calculated for C₄₂H₄₁F₃IN₉O₉: 999.20; Found 1000.4 (M+H).

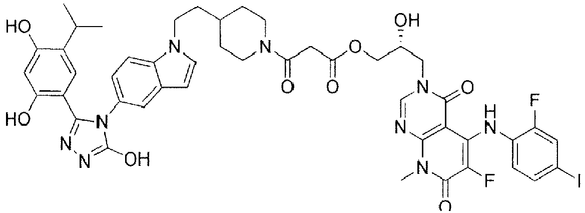

### SDC-TRAP-0532

### (R)-3-(6-fluoro-5-((2-fluoro-4-iodophenyl)amino)-8-methyl-4,7-dioxo-7,8-dihydropyrido[2,3-d]pyrimidin-3(4H)-yl)-2-hydroxypropyl 3-(4-(2-(5-(3-(2,4-dihydroxy-5-isopropylphenyl)-5-hydroxy-4H-1,2,4-triazol-4-yl)-1H-indol-1-yl)ethyl)piperidin-1-yl)-3-oxopropanoate.

Following the general procedure to SDC-TRAP-0524, SDC-TRAP-0532 was prepared; ESMS calculated for C₄₆H₄₆F₂IN₉O₉: 1033.24; Found 1034.0 (M+H).

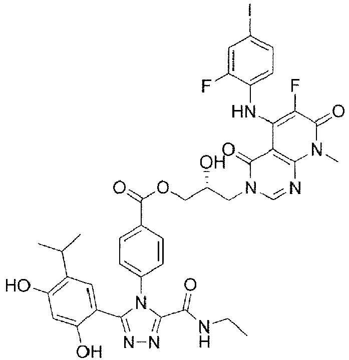

### SDC-TRAP-0533

### (R)-3-(6-fluoro-5-((2-fluoro-4-iodophenyl)amino)-8-methyl-4,7-dioxo-7,8-dihydropyrido[2,3-d]pyrimidin-3(4H)-yl)-2-hydroxypropyl 4-(3-(2,4-dihydroxy-5-isopropylphenyl)-5-(ethylcarbamoyl)-4H-1,2,4-triazol-4-yl)benzoate.

Following the general procedure to SDC-TRAP-0524, SDC-TRAP-0533 was prepared; ESMS calculated (C₃₈H₃₅F₂IN₈O₈): 896.2; found: 897.0 (M+H).

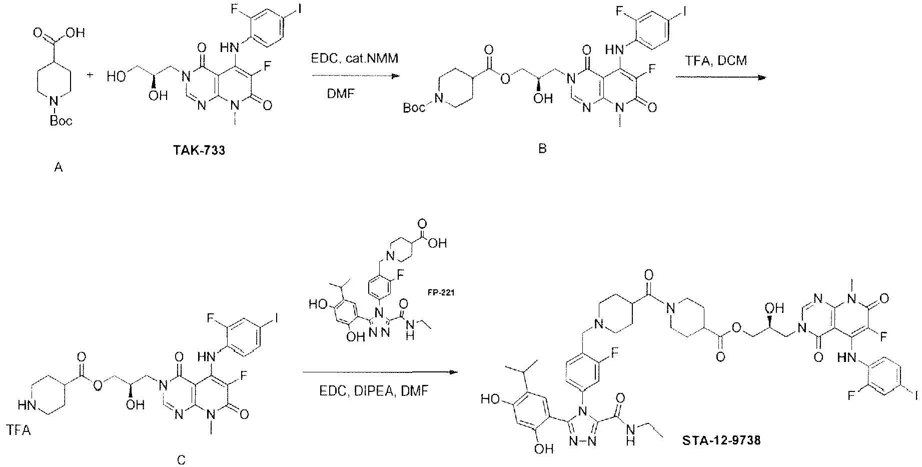

To a solution of *N*-Boc-piperidine-4-carboxylic acid A (0.07 g, 0.3 mmol) and FP-241(150mg, 0.3 mmol) in DMF (3 mL) was added EDC (0.01 g, 0.5 mmol) and NMM (0.01 g, 0.01 mmol). The mixture was stirred overnight at RT. The reaction mixture was diluted with EtOAc, washed with water and saturated aq. NaCl. The organic layer was dried over Na₂SO₄ and concentrated under reduced pressure. The residue was purified by flash column chromatography on silica gel (DMC/MeOH 99:1 to 9:1) to give compound B as white solid (0.07 g, 32.5% yield).

Compound B (0.07 g, 0.097 mmol) was stirred in mixture TFA/DCM (1:3) (4 mL) at 0°C for 2h. TLC indicated that reaction was completed. The mixture was concentrated and dried in vacuum to give 68mg compound C as crude product. To a solution of C (0.068 g, 0.095 mmol) and FP-221 (0.06 g,0.1mmol) in DMF (3 mL) was added EDC (0.04 g, 0.2 mmol) and DIPEA (0.04 mL,0.2 mmol). The mixture was stirred overnight at RT. The reaction mixture was diluted with EtOAc, washed with water and saturated aq. NaCl. The organic layer was dried over Na₂SO₄ and concentrated under reduced pressure. The residue was purified by flash column chromatography on silica gel (DMC/MeOH 98:2 to 9:1) to give compound as white solid (0.045 g, 42% yield).

### SDC-TRAP-0534

(S)-3-(6-fluoro-5-((2-fluoro-4-iodophenyl)amino)-8-methyl-4,7-dioxo-7,8-dihydropyrido[2,3-d]pyrimidin-3(4H)-yl)-2-hydroxypropyl 1-(1-(4-(3-(2,4-dihydroxy-5-isopropylphenyl)-5-(ethylcarbamoyl)-4H-1,2,4-triazol-4-yl)-2-fluorobenzyl)piperidine-4-carbonyl)piperidine-4-carboxylate. ¹H NMR (400 MHz, DMSO) δ 10.35 (s, 1H), 10.19 (s, 1H), 9.79 (s, 1H), 9.01 (s, 1H), 8.84(bs, 1H), 8.55 (s, 1H), 7.70 (s, 1H), 7.52 (d, *J* = 1.6 Hz, 1H), 7.47 (d, *J* = 8.8 Hz, 1H), 7.36 (d, *J* = 1.6 Hz, 1H), 7.20 (d, *J* = 1.6 Hz, 1H), 6.94 (dd, *J* = 8.4, 2.0 Hz, 1H), 6.67(s, 1H), 6.35 (s, 1H), 5.56 (s, 1H), 4.24-4.22(m, 2H),4.06-3.80(m,5H), 3.59 (s, 3H),3.12-2.60(m, 13H), 2.12-1.50 (m, 9H), 1.05 (t, *J* = 4.8 Hz, 3H), 0.87 (d, *J* = 8.8 Hz, 6H); ESMS calculated for C₅₀H₅₄F₃IN₁₀O₉: 1122.31; Found 1123.0 (M+H).

### Example 3- Hsp90 inhibitor Monomethyl auristatin E (MMAE) conjugates

### General procedure for MMAE amide conjugation:

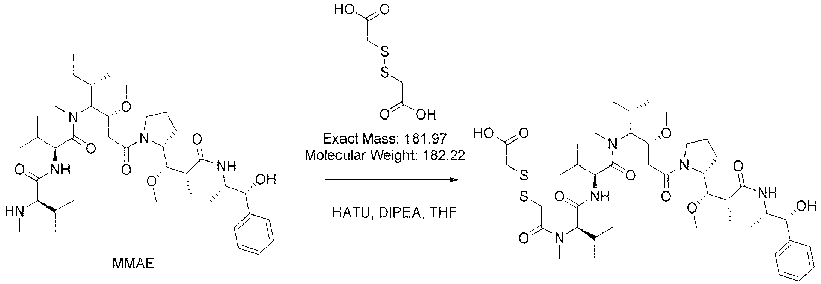

To the solution of MMAE (0.07 g, 0.1 mmol) in THF (3 mL) was added 2,2'-disulfanediyldiacetic acid (0.027 g, 0.15 mmol), HATU (0.045 g, 0.12 mmol), and DIPEA (0.035 mL, 0.2 mmol). The reaction was stirred at room temperature for 5 hr. The solution was concentrated and the column chromatography (DCM/MeOH) gave the product (0.045 mg, 51%).

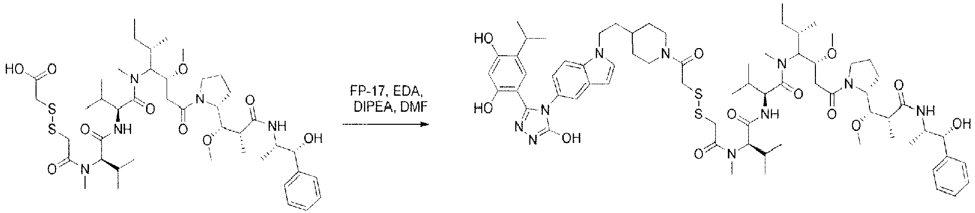

To the solution of above product (0.04 g, 0.045 mmol) in DMF (2 mL) was added SDC-TRAP-0062 (HCl salt, 0.025 g, 0.05 mmol), EDC (0.018 g, 0.09 mmol), and DIPEA (0.016 mL, 0.09 mol). The reaction was stirred at room temperature overnight. The reaction solution was diluted with H₂O (2 mL) and extracted with EtOAc (5 mLX3). The organic phase was separated, dried over Na₂SO₄ and concentrated. The column chromatography (DCM/MeOH) gave SDC-TRAP-0535 (0.057 g, 51%).

### SDC-TRAP-0535

### (2S)-2-((R)-2-(2-((2-(4-(2-(5-(3-(2,4-dihydroxy-5-isopropylphenyl)-5-hydroxy-4H-1,2,4-triazol-4-yl)-1H-indol-1-yl)ethyl)piperidin-1-yl)-2-oxoethyl)disulfanyl)-N-methylacetamido)-3-methylbutanamido)-N-((3R,5S)-1-((R)-2-((1S,2R)-3-(((1R,2S)-1-hydroxy-1-phenylpropan-2-yl)amino)-1-methoxy-2-methyl-3-oxopropyl)pyrrolidin-1-yl)-3-methoxy-5-methyl-1-oxoheptan-4-yl)-N,3-dimethylbutanamide. ESMS calculated (C₆₉H₁₀₀N₁₀O₁₂S₂): 1324.7; found: 1325.2 (M+H).

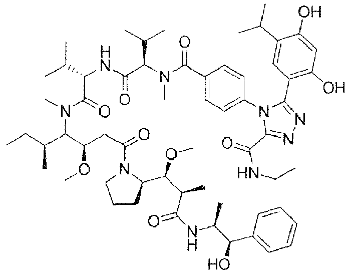

### SDC-TRAP-0536

### 5-(2,4-dihydroxy-5-isopropylphenyl)-N-ethyl-4-(4-(((2R)-1-(((2S)-1-(((3R,5S)-1-((R)-2-((1S,2R)-3-(((1R,2S)-1-hydroxy-1-phenylpropan-2-yl)amino)-1-methoxy-2-methyl-3-oxopropyl) pyrrolidin-1-yl)-3-methoxy-5-methyl-1-oxoheptan-4-yl)(methyl)amino)-3-methyl-1-oxobutan-2-yl)amino)-3-methyl-1-oxobutan-2-yl)(methyl)carbamoyl)phenyl)-4H-1,2,4-triazole-3-carboxamide.

Following the general procedure to SDC-TRAP-0535, SDC-TRAP-0536 was prepared; ¹H NMR (400 MHz, DMSO) δ 10.25 (s, 1H), 9.76 (s, 1 H), 9.03 (s, 1H), 8.08-765(m, 2H), 743-7.26 (m, 8H), 6.70 (s, 1H). 6.32 (s, 1H), 5.44-5.31 (m, 1H), 4.68-3.85(m, 4H), 4.01-3.75 (m, 3H),3.25-2.76(m, 10H), 2.45-1.60 (m, 10H), 1.05-0.79 (m, 25H); ESMS calculated for C₆₀H₈₇N₉O₁₁: 1109.65; Found 1110.2 (M+H).

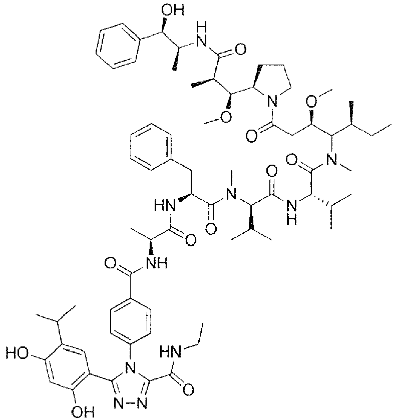

### SDC-TRAP-0537

### 4-(4-(((3R,7S,10R,13S,16S)-13-benzyl-4-((S)-sec-butyl)-3-(2-((R)-2-((1S2R)-3-(((1R,2S)-1-hydroxy-1-phenylpropan-2-yl)amino)-1-methoxy-2-methyl-3-oxopropyl)pyrrolidin-1-yl)-2-oxoethyl)-7,10-diisopropyl-5,11-dimethyl-6,9,12,15-tetraoxo-2-oxa-5,8,11,14-tetraazaheptadecan-16-yl)carbamoyl)phenyl)-5-(2,4-dihydroxy-5-isopropylphenyl)-N-ethyl-4H-1,2,4-triazole-3-carboxamide

Following the general procedure to SDC-TRAP-0535, SDC-TRAP-0537 was prepared; ESMS calculated for C₇₂H₁₀₁N₁₁O₁₃: 1327.76; Found 1328.9 (M+H).

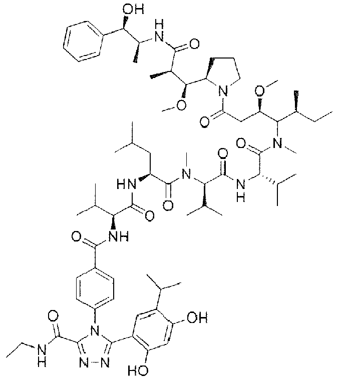

### SDC-TRAP-0538

### 4-(4-(((3R,7S,10R,13S,16S)-4-((S)-sec-butyl)-3-(2-((R)-2-((1S,2R)-3-(((1R,2S)-1-hydroxy-1-phenylpropan-2-yl)amino)-1-methoxy-2-methyl-3-oxopropyl)pyrrolidin-1-yl)-2-oxoethyl)-13-isobutyl-7,10-diisopropyl-5,11,17-trimethyl-6,9,12,15-tetraoxo-2-oxa-5,8,11,14-tetraazaoctadecan-16-yl)carbamoyl)phenyl)-5-(2,4-dihydroxy-5-isopropylphenyl)-N-ethyl-4H-1,2,4-triazole-3-carboxamide

Following the general procedure to SDC-TRAP-0535, SDC-TRAP-0538 was prepared; ESMS calculated for C₇₁H₁₀₇N₁₁O₁₃: 1321.80; Found 1322.9 (M+H).

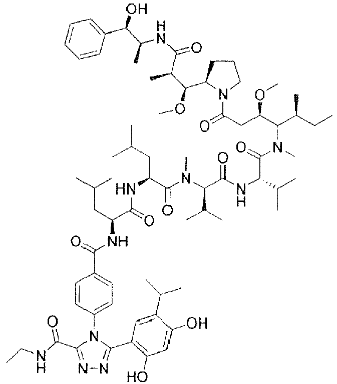

### SDC-TRAP-0539

### 4-(4-(((3R,7S,10R,13S,16S)-4-((S)-sec-butyl)-3-(2-((R)-2-((1S,2R)-3-(((1R,2S)-1-hydroxy-1-phenylpropan-2-yl)amino)-1-methoxy-2-methyl-3-oxopropyl)pyrrolidin-1-yl)-2-oxoethyl)-13-isobutyl-7,10-diisopropyl-5,11,18-trimethyl-6,9,12,15-tetraoxo-2-oxa-5,8,11,14-tetraazanonadecan-16-yl)carbamoyl)phenyl)-5-(2,4-dihydroxy-5-isopropylphenyl)-N-ethyl-4H-1,2,4-triazole-3-carboxamide

Following the general procedure to SDC-TRAP-0535, SDC-TRAP-0539 was prepared; ESMS calculated for C₇₂H₁₀₉N₁₁O₁₃: 1335.82; Found 1336.9 (M+H).

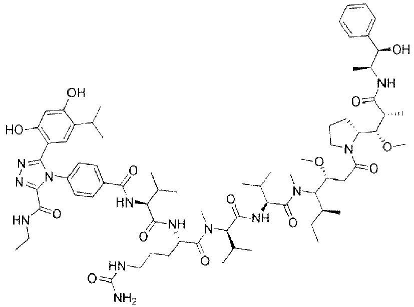

### SDC-TRAP-0540

### 4-(4-(((3R,7S,10R,13S,16S)-4-((S)-sec-butyl)-3-(2-((R)-2-((1S,2R)-3-(((1R,2S)-1-hydroxy-1-phenylpropan-2-yl)amino)-1-methoxy-2-methyl-3-oxopropyl)pyrrolidin-1-yl)-2-oxoethyl)-7,10-diisopropyl-5,11,17-trimethyl-6,9,12,15-tetraoxo-13-(3-ureidopropyl)-2-oxa-5,8,11,14-tetraazaoctadecan-16-yl)carbamoyl)phenyl)-5-(2,4-dihydroxy-5-isopropylphenyl)-N-ethyl-4H-1,2,4-triazole-3-carboxamide.

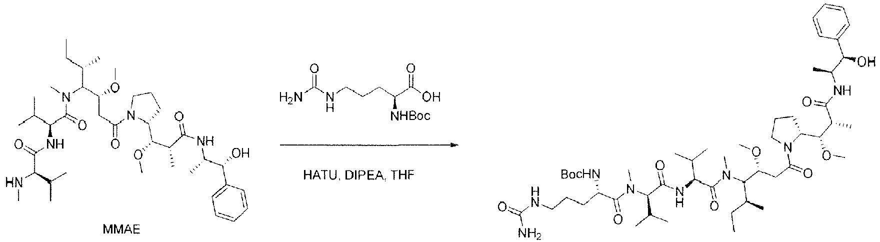

To the solution of MMAE (0.07 g, 0.1 mmol) in THF (3 mL) was added Boc-Cit-OH acid (0.041 g, 0.15 mmol), HATU (0.045 g, 0.12 mmol), and DIPEA (0.035 mL, 0.2 mmol). The reaction was stirred at room temperature overnight. The solution was concentrated and the column chromatography (DCM/MeOH) gave the product (0.062 mg, 64%).

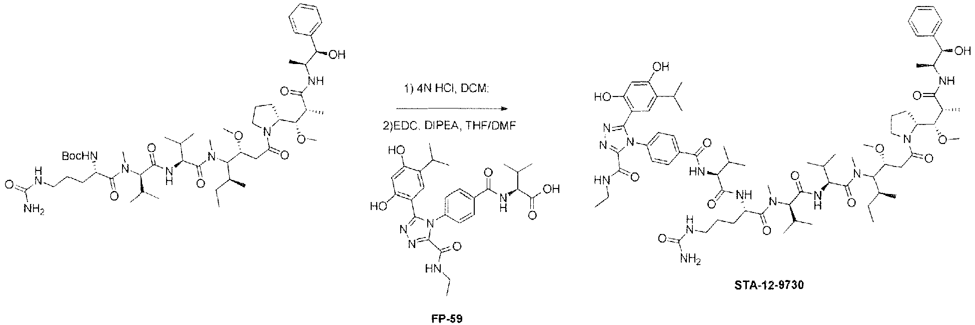

To the solution of above product (0.06 g, 0.06 mmol) in DCM (3.6 mL) was added HCl (4N, in dioxane, 0.4 mL). The solution was stirred at room temperature for 1 hr before it was concentrated. The resulting crude product was dissolved in THF/DMF (3mL/lmL). To this solution was added FP-59 (0.03 g, 0.06 mmol), EDC (0.023 g, 0.12 mmol), and DIPEA (0.03 mL, 0.18 mmol). The reaction was stirred at room temperature for 2 hr. The reaction solution was diluted with H₂O (2 mL) and extracted with EtOAc (5 mLX3). The organic phase was separated, dried over Na₂SO₄ and concentrated. The column chromatography (DCM/MeOH) gave SDC-TRAP-0540 (0.025 g, 31%). ESMS calculated (C₇₁H₁₀₇N₁₃O₁₄): 1365.8; found: 1366.5 (M+H).

### Example 4

The HDCs as described herein were used in various experiments to determine their activity and properties, such as binding affinity and stability in blood. An in-Cell Western assay (ICW) for HER2 expression was carried out in BT-474 human breast cancer cells. BT-474 cells were seeded in 96-well plates at a density of 20,000 cells per well. Twenty-four hours after plating, cells were dosed with graded concentrations of HDCs using 3-fold serial dilutions and incubated for 24 h. Growth media containing drug was aspirated off. Cells were then fixed in 4 % paraformaldehyde for 20 min and washed three times in 0.1% Triton X-100. After blocking for 1.5 h in Odyssey Blocking Buffer (LI-COR, Lincoln, NE, USA), cells were incubated with primary HER2 antibody overnight at 4 °C. Wells were washed three times with 0.1 % Tween-20 and incubated in secondary antibody (LI-COR, Lincoln, NE, USA) and DRAQ5 (CST, Beverly, MA, USA) for 1 h. Following 3 final washes with 0.1 % Tween-20, antibody-antigen complexes were visualized using the Odyssey system. For quantification, HER2 signal was normalized to DRAQ5 flourescent probe. The results are shown below in Table 1.

**Table 1**

| **No** | **SDC-TRAP #** | **Payload** | **HER2 (ICW, nM)** |
|---|---|---|---|
| ganetespib | | | 37 |
| 1 | 0507 | BVD-523 | 304 |
| 2 | 0510 | BVD-523 | 91 |
| 3 | 0511 | BVD-523 | 465 |
| 4 | 0512 | BVD-523 | 60 |
| 5 | 0513 | BVD-523 | 519 |
| 6 | 0508 | BVD-523 | 2334 |
| 7 | 0509 | BVD-523 | 2689 |
| 8 | 0514 | BVD-523 | 345 |
| 9 | 0515 | BVD-523 | 181 |
| 10 | 0516 | BVD-523 | 123 |
| 11 | 0519 | BVD-523 | 1649 |
| 12 | 0518 | BVD-523 | 104 |
| 13 | 0517 | BVD-523 | 202 |

Metabolic stability of various HDCs was tested in mouse blood. All compounds were tested at a concentration of 10 µM. The time dependent disappearance of test compound in mouse whole blood, and the release of the compound qualitatively as determined by liquid chromatography tandem mass spectrometry (LC-MS/MS), was tested at time points of 15 minutes, 30 minutes, 45 minutes and 1 hour. All compounds were tested in duplicate, and the average was taken. The results in Table 2 show the percent of test compound remaining after 1 hour.

**Table 2**

| **No** | **SDC-TRAP #** | **Payload** | **Mouse blood stability** |
|---|---|---|---|
| ganetespib | | | **1h (% remaining)** |
| 1 | 0507 | BVD-523 | 105% |
| 2 | 0510 | BVD-523 | 77.1% |
| 3 | 0511 | BVD-523 | 30.8% |
| 4 | 0512 | BVD-523 | 100% |
| 5 | 0513 | BVD-523 | 48.2% |
| 6 | 0508 | BVD-523 | 32.4% |
| 7 | 0509 | BVD-523 | 86.9% |
| 8 | 0514 | BVD-523 | 105% |
| 9 | 0515 | BVD-523 | 30% |
| 10 | 0516 | BVD-523 | 55.5% |
| 20 | 0525 | TAK-733 | 10.8% |
| 23 | 0528 | TAK-733 | 0% |
| 28 | 0538 | MMAE | 118% |
| 29 | 0539 | MMAE | 106% |
| 30 | 0540 | MMAE | 55.5% |

Finally, binding affinity (Kd) was determined for various HDCs using standard methods. The results are shown in Table 3.

**Table 3**

| **No** | **SDC-TRAP#** | **Payload** | **Affinity (Kd, nM)** |
|---|---|---|---|
| Ganetespib | | | 0.13 |
| 2 | 0510 | BVD-523 | 6.14 |
| 3 | 0511 | BVD-523 | 2.90 |
| 5 | 0513 | BVD-523 | 0.88 |
| 9 | 0515 | BVD-523 | 0.97 |
| 10 | 0516 | BVD-523 | 1.29 |
| 26 | 0536 | MMAE | 0.77 |

All publications, patent applications, patents, and other documents cited herein are incorporated by reference in their entirety. In case of conflict, the present specification, including definitions, will control.

The specification should be understood as disclosing and encompassing all possible permutations and combinations of the described aspects, embodiments, and examples unless the context indicates otherwise. One of ordinary skill in the art will appreciate that the invention can be practiced by other than the summarized and described aspect, embodiments, and examples, which are presented for purposes of illustration, and that the invention is limited only by the following claims.

Further embodiments of the present invention are the following:
1. An Hsp90 drug conjugate (HDC) comprising an Hsp90 ligand or prodrug thereof, a linker moiety and a payload moiety.
2. The HDC of clause 1, wherein the Hsp90 ligand is an Hsp90 inhibitor.
3. The HDC of clause 2, wherein the Hsp90 inhibitor is ganetespib, AUY-922 or AT-13387.
4. The HDC of clause 1, wherein the payload moiety is selected from the group consisting of: a therapeutic moiety, a cytotoxic moiety and an imaging moiety.
5. The HDC of clause 4, wherein the therapeutic moiety is a cytotoxic moiety.
6. The HDC of clause 4, wherein the therapeutic moiety is a chemotherapeutic agent.
7. The HDC of clause 5, wherein the cytotoxic moiety is an auristatin.
8. The HDC of clause 7, wherein the auristatin is Monomethyl auristatin E (MMAE).
9. The HDC of clause 8, wherein the HDC is selected from the group consisting of SDC-TRAP-0535 to SDC-TRAP-0540.
10. The HDC of clause 6, wherein the chemotherapeutic agent is selected from the group consisting of: imids, proteasome inhibitors, PARP inhibitors, cell cycle inhibitors, alkylating agents, anthracyclines, antimetabolites, epigenetic modifiers, hormonal therapy, microtubule stabilizers, platinums and tyrosine kinase inhibitors.
11. The HDC of clause 6, wherein the chemotherapeutic agent is an ERK kinase inhibitor.
12. The HDC of clause 11, wherein the ERK kinase inhibitor is BVD-523.
13. The HDC of clause 12, wherein the HDC is selected from the group consisting of SDC-TRAP-0507 to SDC-TRAP-0523.
14. The HDC of clause 6, wherein the chemotherapeutic agent is a MEK inhibitor.
15. The HDC of clause 14, wherein the MEK inhibitor is TAK-733.
16. The HDC of clause 15, wherein the HDC is selected from the group consisting of SDC-TRAP-0524 to SDC-TRAP-0534.
17. The HDC of clause 5, wherein the cytotoxic moiety is not suitable for administration alone.
18. The HDC of clause 1, wherein the linker moiety and the payload moiety are covalently attached.
19. The HDC of clause 1, wherein the Hsp90 ligand and the linker moiety are covalently attached.
20. The HDC of clause 1, wherein the linker moiety is a cleavable linker.
21. The HDC of clause 20, wherein the cleavable linker comprises an enzymatically cleavable linker.
22. The HDC of clause 1, wherein the linker is a disulfide linker.
23. The HDC of clause 1, wherein the linker is a dipeptide linker.
24. The HDC of clause 23, wherein the dipeptide linker is a valine-citrulline (val-cit) linker or a valine-citrulline p-aminobenzylcarbamate (val-cit-PABC) linker.
25. The HDC of clause 1, wherein the HDC is able to enter a cell by passive diffusion.
26. The HDC of clause 1, wherein the Hsp90 ligand or prodrug thereof inhibits Hsp90.
27. The HDC of clause 1, wherein the Hsp90 ligand or prodrug thereof does not inhibit Hsp90.
28. A method of treating a disease or disorder in a subject, comprising administering to the subject an effective amount of an HDC of any of the previous claims.
29. A method of treating cancer in a subject, comprising administering to the subject an effective amount of an HDC of any of the previous claims.
30. The method of clause 29, wherein the method further comprises administering an additional anticancer therapy.
31. A kit comprising an HDC, wherein the HDC comprises an Hsp90 ligand or prodrug thereof, a linker moiety and a payload moiety, and instructions for use in treating a disease or disorder.
32. The kit of clause 31, wherein the disease or disorder is cancer.

## Claims

1. An Hsp90 drug conjugate (HDC) comprising an Hsp90 ligand or prodrug thereof, a linker moiety and a payload moiety, wherein the payload moiety is an ERK kinase inhibitor.

2. The HDC of claim 1, wherein the Hsp90 ligand is an Hsp90 inhibitor,

3. The HDC of claim 2, wherein the Hsp90 inhibitor is ganetespib, AUY-922 or AT-13387.

4. The HDC of claim 1, wherein the ERK kinase inhibitor is BVD-523.

5. The HDC of claim 4, wherein the HDC is selected from the group consisting of SDC-TRAP-0507 to SDC-TRAP-0523.

6. The HDC of claim 1, wherein the linker moiety and the payload moiety are covalently attached and wherein the Hsp90 ligand and the linker moiety are covalently attached.

7. The HDC of claim 1, wherein the linker moiety is a cleavable linker.

8. The HDC of claim 7, wherein the cleavable linker comprises an enzymatically cleavable linker; or
wherein the linker is a disulfide linker; or
wherein the linker is a dipeptide linker, wherein the dipeptide linker preferably is a valine-citrulline (val-cit) linker or a valine-citrulline p-aminobenzylcarbamate (val-cit-PABC) linker.

9. The HDC of claim 1, wherein the Hsp90 ligand or prodrug thereof inhibits Hsp90.

10. The HDC of claim 1, wherein the Hsp90 ligand or prodrug thereof does not inhibit Hsp90.

11. Effective amount of an HDC of any one of the previous claims for use in treating a disease or disorder in a subject; or for use in treating cancer in a subject, wherein the use in treating cancer in a subject preferably further comprises an additional anticancer therapy.

12. A kit comprising an HDC of any one of the previous claims, and instructions for use in treating a disease or disorder, wherein the disease or disorder preferably is cancer.
